# EUROPÄISCHE PATENTANMELDUNG

(11) **EP 3 943 483 A1**
(43) Veröffentlichungstag der Anmeldung: **26.01.2022**
(21) Anmeldenummer: 21176058.2
(22) Anmeldetag: 26.05.2021
(51) Int. Cl.: C07C 209/74, C07C 211/45

(54) **VERFAHREN ZUR HERSTELLUNG VON SUBSTITUIERTEN ANILINEN**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein Verfahren zur Herstellung von Verbindungen der Formel (I) 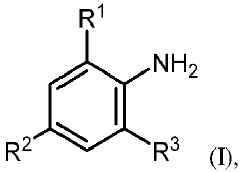 ausgehend von Verbindungen der Formel (IIa) 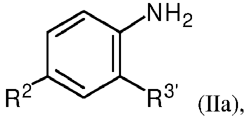 worin R¹, R² , R³ und R^{3'} die erfindungsgemäß beschriebenen Bedeutungen haben.

## Beschreibung

Die vorliegende Erfindung betrifft ein Verfahren zur Herstellung von Verbindungen der Formel (I) 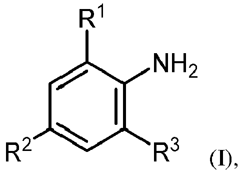 ausgehend von Verbindungen der Formel (IIa) 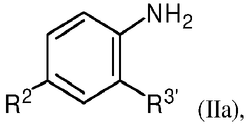 worin R¹, R² , R³ und R^{3'} die nachfolgend beschriebenen Bedeutungen haben.

Ein mögliches Verfahren zur Herstellung von Verbindungen gemäß Formel (I) oder deren Vorstufen ist beispielsweise in EP1380568 und WO2016/174052 beschrieben. Die Herstellung erfolgt durch Perfluoralkylierung in *para* Stellung von in *ortho-* und meta-Stellung bereits substituierten Anilinen. Die beschriebenen Verfahren weisen den Nachteil auf, dass die Produkte in Abhängigkeit der Substitution in schwankenden, zum Teil nur moderaten Ausbeuten erhalten werden oder in guten Ausbeuten ausschließlich mittels sehr Abfall-lastiger Fenton-Oxidation erhalten werden können. Zudem müssen die Verbindungen der Formel (I) in mehrstufigen Prozessen hergestellt werden. Weitere mögliche Verfahren zur Herstellung von Verbindungen der Formel (I) sind ebenfalls in der WO2016/174052 und auch in US2010/0204504, EP2319830 und EP2325165 beschrieben. Dabei werden in einem zweistufigen Verfahren zunächst in para-Stellung perfluoralkylierte Aniline, welche optional auch in *ortho* Stellung substituiert sein können, hergestellt und isoliert. Diese können dann in einem weiteren Schritt in *meta* oder in *meta-* und *ortho-Stellung* zu Verbindungen der Formel (I) halogeniert werden. Nachteilig an den beschriebenen Verfahren ist insbesondere, dass die perfluoralkylierten Intermediate aufgrund ihrer Struktur leicht zur Zersetzung durch Polymerisation neigen und somit nur eine begrenzte Stabilität in konzentrierter Form aufweisen. Bei der Halogenierung der in *para*-Stellung perfluoralkylierten Aniline im zweistufigen Verfahren kann auch eine unerwünschte Polymerisationsnebenreaktion auftreten. Dies führt zu Ausbeuteverlusten und erfordert aufwendige Reinigungsverfahren zur Herstellung von Verbindungen mit hoher Reinheit.

Substituierte Aniline der Formel (I) haben eine große Bedeutung als Baustein zur Synthese neuer Wirkstoffe in der Agrochemie und Tiergesundheit. Die Aufgabe der vorliegenden Erfindung bestand daher darin, ein Verfahren zur Herstellung von Verbindungen der allgemeinen Formel (I) bereitzustellen, das großtechnisch und kostengünstig, insbesondere in einem GMP-regulierten Umfeld, eingesetzt werden kann und die oben beschriebenen Nachteile umgeht. Auch ist es erstrebenswert, die Verbindungen der Formel (I) sowie etwaige Zwischenprodukte mit hoher Ausbeute, hoher Selektivität und hoher Reinheit zu erhalten, so dass die Zielverbindungen vorzugsweise keiner weiteren, eventuell aufwendigen, Aufreinigung unterzogen werden müssen und in einem GMP-regulierten Umfeld eingesetzt werden können.

Diese Aufgabe wurde erfindungsgemäß gelöst, durch ein Verfahren zur Herstellung von Verbindungen der Formel (I) 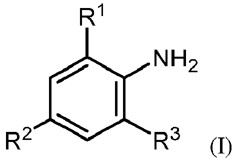 worin
- R¹: für Chlor oder Brom steht,
- R²: für C₁-C₄-Halogenalkyl steht und
- R³: für Cyano, Halogen, gegebenenfalls mit Halogen oder CN substituiertes C₁-C₄-Alkyl oder für gegebenenfalls mit Halogen substituiertes C₁-C₄-Alkoxy steht,
ausgehend von Verbindungen der Formel (IIa), 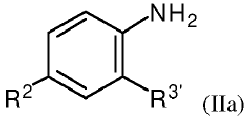 worin R² die oben genannte Bedeutung hat und
R^{3'} für Wasserstoff, Cyano, Halogen, gegebenenfalls mit Halogen oder CN substituiertes C₁-C₄-Alkyl oder für gegebenenfalls mit Halogen substituiertes C₁-C₄-Alkoxy steht, umfassend die folgenden Schritte (1b) und (2)
(1b) Umsetzung von Verbindungen der Formel (IIa) mit einer Verbindung der Formel R⁴SO₃H zu Verbindungen der Formel (III) oder mit einer aromatischen Carbonsäure R⁵CO₂H zu Verbindungen der Formel (IIIa), 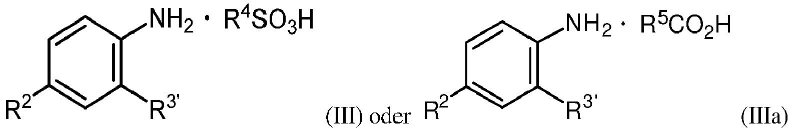 wobei R² und R^{3'} die oben genannten Bedeutungen haben und R⁴ ein gegebenenfalls substituiertes C₁-C₁₂-Alkyl, das verzweigt oder unverzweigt sein kann, oder ein gegebenenfalls substituierter 6- bis 14-gliedriger Aromat ist und R⁵ ein gegebenenfalls substituierter 6-gliedriger Aromat, insbesondere C₆H₅, C₆H₄Cl oder C₆H₄NO₂, ist,
(2) Chlorierung oder Bromierung von Verbindungen der Formel (III) oder (IIIa) mit einem Chlorierungs- oder Bromierungsmittel zu Verbindungen der Formel (I).

Das erfindungsgemäße Verfahren hat gegenüber den vorab beschriebenen Verfahren den Vorteil, dass die gewünschten Verbindungen der Formel (I) in hohen Ausbeuten, mit hoher Selektivität und mit besonders hohen Reinheiten auf einfache Weise ohne aufwendige Aufarbeitungsverfahren erhalten werden. Die so erhaltenen sehr reinen Verbindungen der Formel (I) können dadurch einfacher in einem GMP-regulierten Umfeld integriert werden. Gleichzeitig kann der Gesamtprozess einfacher sowie effizienter und somit kostengünstiger betrieben werden. Außerdem hat sich überraschenderweise herausgestellt, dass bei der Chlorierung oder Bromierung in Schritt (2), von Verbindungen der Formel (III), eine unerwünschte Polymerisation zumindest teilweise unterdrückt werden kann.

Vorteilhafte Ausgestaltungen der Erfindung sind in den abhängigen Ansprüchen angegeben und werden nachfolgend im Einzelnen erläutert. Die im Folgenden beschriebenen bevorzugten Ausführungsformen beziehen sich, wenn zutreffend, auf alle hierin beschriebenen Formeln.

Der Begriff "Halogen" steht im Rahmen dieser Erfindung bevorzugt für Chlor, Fluor, Brom oder Iod.

Sofern nicht an anderer Stelle anders definiert, wird unter dem Begriff "Alkyl", erfindungsgemäß entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12, bevorzugt 1 bis 6, besonders bevorzugt 1 bis 4, ganz besonders bevorzugt 1 bis 3 Kohlenstoffatomen verstanden, die verzweigt oder unverzweigt sein kann.

Beispiele für C₁-C₁₂-Alkylreste sind Methyl, Ethyl, n-Propyl, iso-Propyl, n-Butyl, iso-Butyl, sek-Butyl, tert-Butyl, n-Pentyl, iso-Pentyl, Neopentyl, tert-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die obenstehende Bedeutung aufweist.

Sofern nicht an anderer Stelle anders definiert, wird unter dem Begriff "Aromat" oder "Aryl" erfindungsgemäß ein aromatischer Rest mit 6 bis 14 Kohlenstoffatomen, vorzugsweise Phenyl, Tolyl, insbesondere 4-Tolyl, Naphthyl, Anthryl oder Phenanthrenyl, besonders bevorzugt Phenyl, Tolyl oder 4-Tolyl, verstanden.

Durch Halogen substituierte Reste, z.B. Halogenalkyl (=Haloalkyl) oder Halogenaryl (=Halogenaren), sind einfach oder mehrfach bis zur maximal möglichen Substituentenzahl halogenierte Reste. Bei mehrfacher Halogenierung können die Halogenatome gleich oder verschieden sein. Gegebenenfalls substituierte Reste können, wenn nichts Anderes erwähnt ist, einfach oder mehrfach substituiert sein, wobei bei Mehrfachsubstitutionen die Substituenten gleich oder verschieden sein können.

Durch Cyano substituierte Reste, z.B. substituierte Alkyl- oder Arylreste, sind einfach oder mehrfach bis zur maximal möglichen Substituentenzahl substituierte Reste. Gegebenenfalls substituierte Reste können, wenn nichts Anderes erwähnt ist, einfach oder mehrfach substituiert sein.

Durch Hydroxy substituierte Reste, z.B. substituierte Alkyl- oder Arylreste, sind einfach oder mehrfach bis zur maximal möglichen Substituentenzahl substituierte Reste. Gegebenenfalls substituierte Reste können, wenn nichts Anderes erwähnt ist, einfach oder mehrfach substituiert sein.

Vorzugsweise wird im erfindungsgemäßen Verfahren in Schritt (1b) eine Verbindung der Formel R⁴SO₃H eingesetzt.

Der Begriff "R⁴SO₃H" steht für eine gegebenenfalls substituierte Sulfonsäure. Dabei kann R⁴ für ein gegebenenfalls mit Halogen, Cyano oder Hydroxy substituiertes C₁-, C₂-, C₃-, C₄-, C₅-, C₆-, C₇-, C₈-, C₉-, C₁₀-, C₁₁- oder C₁₂-Alkyl, das verzweigt oder unverzweigt sein kann, stehen. Dabei kann R⁴ auch für einen 6- bis 14- gliedrigen Aromaten stehen, der gegebenenfalls mit Halogen, Cyano oder Hydroxy oder mit einem gegebenenfalls mit Halogen, Cyano oder Hydroxy substituiertem C₁-C₁₂-Alkyl, das verzweigt oder unverzweigt sein kann, substituiert ist.

Der Begriff "R⁵CO₂H" steht für eine gegebenenfalls substituierte Benzoesäure. R⁵ ist ein 6-gliedriger Aromat, der gegebenenfalls mit Halogen, Cyano oder Hydroxy oder mit einem gegebenenfalls mit Halogen, Cyano oder Hydroxy substituiertem C₁-C₁₂-Alkyl, das verzweigt oder unverzweigt sein kann, substituiert sein kann. R⁵CO₂H ist bevorzugt C₆H₅CO₂H, ClC₆H₄CO₂H oder HCO₂CC₆H₄NO₂.

In einer bevorzugten Ausführungsform der Erfindung steht
- R¹: für Halogen.

Besonders bevorzugt steht
- R¹: für Chlor, Fluor, Brom oder Iod.

Ganz besonders bevorzugt steht
- R¹: für Chlor, Fluor oder Brom.

In einer bevorzugten Ausführungsform der Erfindung steht
- R²: für mit Fluor substituiertes C₁-C₄-Alkyl.

Besonders bevorzugt steht
- R²: für Perfluoro-C₁-C₃-Alkyl (CF₃, C₂F₅ oder C₃F₇ (n- oder iso-Propyl)).

Ganz besonders bevorzugt steht
- R²: für Heptafluoro-iso-propyl.

In einer weiteren bevorzugten Ausführungsform steht
- R³: für einen Substituenten ausgewählt aus Cl, Br, F, C₁-C₃-Alkyl, mit Halogen substituiertes C₁-C₃-Alkyl, C₁-C₃-Alkoxy oder mit Halogen substituiertes C₁-C₃-Alkoxy.

In einer besonders bevorzugten Ausführungsform steht
R³ für Cl, Br, C₁-C₃-Alkyl oder mit Fluor substituiertes C₁-C₃-Alkyl, C₁-C₃-Alkoxy oder mit Fluor substituiertes C₁-C₃-Alkoxy.

Ganz besonders bevorzugt steht
- R³: für Cl, Trifluormethyl, Trifluormethoxy oder Difluormethoxy.

In einer weiteren bevorzugten Ausführungsform steht
R⁴ für ein gegebenenfalls mit Halogen, Cyano oder Hydroxy substituiertes C₁-C₁₂-Alkyl, das verzweigt oder unverzweigt sein kann, oder für einen 6- bis 14-gliedrigen Aromaten, der gegebenenfalls mit Halogen, Cyano oder Hydroxy oder mit einem gegebenenfalls mit Halogen, Cyano oder Hydroxy substituiertem C₁-C₁₂-Alkyl, das verzweigt oder unverzweigt sein kann, substituiert ist.

In einer besonders bevorzugten Ausführungsform steht
R⁴ für ein gegebenenfalls mit Halogen, Cyano oder Hydroxy substituiertes C₁-C₄-Alkyl, das verzweigt oder unverzweigt sein kann, oder einen 6-gliedrigen Aromat, der gegebenenfalls mit Halogen, Cyano oder Hydroxy oder mit einem gegebenenfalls mit Halogen, Cyano oder Hydroxy substituiertem C₁-C₁₂-Alkyl, das verzweigt oder unverzweigt sein kann, substituiert ist.

Ganz besonders bevorzugt steht
- R⁴: für Methyl-, Tolyl-, insbesondere 4-Tolyl, oder Phenyl.

Am meisten bevorzugt steht
- R⁴: für 4-Tolyl.

In einer besonders vorteilhaften Ausgestaltung der Erfindung stehen R¹ und R³ beide für Chlor oder Brom, insbesondere bevorzugt für Chlor.

In einer weiteren besonders vorteilhaften Ausgestaltung der Erfindung steht
R¹ für Chlor oder Brom,
R² für Perfluoro-C₁-C₃-Alkyl,
R³ für Halogen, C₁-C₃-Alkyl oder mit Fluor substituiertes C₁-C₃-Alkyl, C₁-C₃-Alkoxy oder mit Fluor substituiertes C₁-C₃-Alkoxy und
R⁴ für Methyl-, Tolyl-, insbesondere 4-Tolyl, oder Phenyl.

In einer ganz besonders vorteilhaften Ausgestaltung der Erfindung steht
R¹ für Chlor oder Brom,
R² für Heptafluor-iso-propyl,
R³ für Cl, Trifluormethyl, Trifluormethoxy oder Difluormethoxy und
R⁴ für 4-Tolyl oder Phenyl.

R⁴SO₃H ist vorzugsweise Methansulfonsäure, 4-Toluolsulfonsäure oder Benzensulfonsäure.

In einer weiteren bevorzugten Ausführungsform steht
R^{3'} für einen Substituenten ausgewählt aus Wasserstoff, Cl, Br, F, C₁-C₃-Alkyl, mit Halogen substituiertes C₁-C₃-Alkyl, C₁-C₃-Alkoxy oder mit Halogen substituiertes C₁-C₃-Alkoxy.

In einer besonders bevorzugten Ausführungsform steht
R^{3'} für Wasserstoff, Cl, Br, C₁-C₃-Alkyl oder mit Fluor substituiertes C₁-C₃-Alkyl, C₁-C₃-Alkoxy oder mit Fluor substituiertes C₁-C₃-Alkoxy.

Ganz besonders bevorzugt steht
- R^{3'}: für Wasserstoff, Cl, Trifluormethyl, Trifluormethoxy oder Difluormethoxy.

Für die Verbindungen der Formel (II) kommen verschiedene Verbindungen in Frage. Verbindungen der Formel (II) werden hierin auch als "Aniline der Formel (II)" bezeichnet. Als Aniline der Formel (II) werden vorzugsweise kommerziell erhältliche Verbindungen eingesetzt.

Bevorzugt kommen die folgenden Verbindungen als Aniline der Formel (II) in Frage:
Anilin,
2-Methylanilin,
2-Chloranilin,
2-Trifluormethylanilin,
2-Trifluormethoxyanilin und
2-Difluormethoxyanilin.

Besonders bevorzugt sind dabei die folgenden Verbindungen:
Anilin,
2-Chloranilin,
2-Trifluormethylanilin,
2-Trifluormethoxyanilin und
2-Difluormethoxyanilin.

Aus diesen Verbindungen ergeben sich bevorzugt die folgenden Verbindungen der Formel (I):
2,6-Dichlor-4-(1,1,1,2,3,3,3-heptafluorpropan-2-yl)anilin,
2-Chlor-6-methyl-4-(1,1,1,2,3,3,3-heptafluorpropan-2-yl)anilin,
2-Brom-6-methy1-4-(1,1,1,2,3,3,3-heptafluorpropan-2-yl)anilin,
2-Chlor-4-(1,1,1,2,3,3,3-heptafluorpropan-2-yl)-6-(trifluormethyl)anilin,
2-Chlor-4-(1,1,1,2,3,3,3-heptafluorpropan-2-yl)-6-(trifluormethoxy)anilin,
2-Chlor-6-(difluormethoxy)-4-(1,1,1,2,3,3,3-heptafluorpropan-2-yl)anilin,
2-Brom-4-(1,1,1,2,3,3,3-heptafluorpropan-2-yl)-6-(trifluormethyl)anilin und
2-Brom-4-(1,1,1,2,3,3,3-heptafluorpropan-2-yl)-6-(trifluormethoxy)anilin.

Besonders bevorzugt sind
2,6-Dichlor-4-(1,1,1,2,3,3,3-heptafluorpropan-2-yl)anilin,
2-Chlor-4-(1,1,1,2,3,3,3-heptafluorpropan-2-yl)-6-(trifluormethyl)anilin,
2-Chlor-4-(1,1,1,2,3,3,3-heptafluorpropan-2-yl)-6-(trifluormethoxy)anilin,
2-Chlor-6-(difluormethoxy)-4-(1,1,1,2,3,3,3-heptafluorpropan-2-yl)anilin und
2-Brom-4-(1,1,1,2,3,3,3-heptafluorpropan-2-yl)-6-(trifluormethoxy)anilin.

Die allgemeinen oder in Vorzugsbereichen aufgeführten Bereiche gelten für das Gesamtverfahren entsprechend. Diese Definitionen können untereinander, also auch zwischen den jeweiligen Vorzugsbereichen, beliebig kombiniert werden.

Erfindungsgemäß bevorzugt verwendet werden Verfahren, in welchen eine Kombination der vorstehend als bevorzugt aufgeführten Bedeutungen und Bereiche vorliegt.

Erfindungsgemäß besonders bevorzugt verwendet werden Verfahren, in welchen eine Kombination der vorstehend als besonders bevorzugt aufgeführten Bedeutungen und Bereiche vorliegt.

Erfindungsgemäß ganz besonders bevorzugt verwendet werden Verfahren, in welchen eine Kombination der vorstehend als ganz besonders bevorzugt aufgeführten Bedeutungen und Bereiche vorliegt.

Erfindungsgemäß insbesondere verwendet werden Verfahren, in welchen eine Kombination der vorstehend als insbesondere aufgeführten Bedeutungen und Bereiche vorliegt.

Erfindungsgemäß hervorgehoben verwendet werden Verfahren, in welchen eine Kombination der vorstehend als hervorgehoben aufgeführten Bedeutungen und Bereiche vorliegt.

### Verfahrensbeschreibung

### Herstellung von Verbindungen der Formel (III) oder (IIIa):

In Schritt (1b) werden erfindungsgemäß Verbindungen der Formel (IIa) mit einer Verbindung der Formel R⁴SO₃H zu Verbindungen der Formel (III) oder mit einer aromatischen Carbonsäure R⁵CO₂H zu Verbindungen der Formel (IIIa), vorzugsweise mit einer Verbindung der Formel R⁴SO₃H zu Verbindungen der Formel (III), umgesetzt. Die Verbindungen der Formel (IIa) können kommerziell erworben werden oder sie können in einem weiteren Schritt hergestellt werden. Insbesondere können die Verbindungen der Formel (IIa) in einem weiteren Schritt (1a) hergestellt werden durch Umsetzung von Verbindungen der Formel (II) 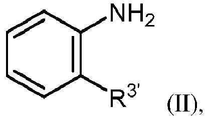 mit Verbindungen der Formel R²-Y, wobei Y für Iod oder Brom steht, und wobei R² und R^{3'} die oben genannten Bedeutungen haben. Die in Schritt (1a) erhaltenen Verbindungen können in Schritt (1b) mit einer Verbindung der Formel R⁴SO₃H zu Verbindungen der Formel (III) oder mit einer aromatischen Carbonsäure R⁵CO₂H zu Verbindungen der Formel (lila), vorzugsweise mit einer Verbindung der Formel R⁴SO₃H zu Verbindungen der Formel (III), umgesetzt werden, 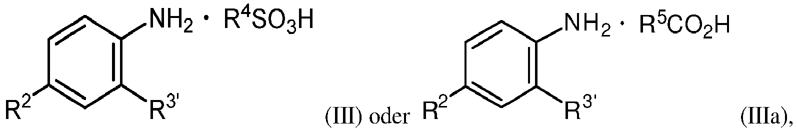 wobei R², R^{3'}, R⁴ und R⁵ die oben genannten Bedeutungen haben.

In einer bevorzugten Ausführungsform der Erfindung steht
- Y: für Iod.

Bevorzugte Verbindungen der Formel R²-Y sind insbesondere Pentafluoroiodethan, Heptafluoro-1-iodpropan, Heptafluoro-2-iod-propan und Heptafluoro-2-brom-propan, besonders bevorzugt sind Heptafluoro-2-iod-propan und Heptafluoro-2-brom-propan, ganz besonders bevorzugt ist Heptafluoro-2-iod-propan.

Die Herstellung der Produkte in Schritt (1a) kann beispielsweise in Analogie zu den in JP 2012/153635 A und CN 106748807 A und US 9 237 745 beschriebenen Methoden aus den entsprechenden Anilinen erfolgen.

In Schritt (1a) werden bevorzugt 0,9 bis 2,0 Äquivalente, besonders bevorzugt 1,0 bis 1,8 Äquivalente, ganz besonders bevorzugt 1,0 bis 1,5 Äquivalente, bezogen auf die gesamte eingesetzte Stoffmenge der Verbindungen der Formel (II), der Verbindungen der Formel R²-Y eingesetzt. Die Verwendung größerer Überschüsse ist zwar chemisch möglich jedoch aus wirtschaftlicher Sicht nicht sinnvoll.

Die Verbindungen der Formel R²-Y werden dabei bevorzugt als Reinstoff, oder als Lösung in dem für die Reaktion bevorzugten Lösungsmittel in Konzentrationen von 40-95 Gew.%, besonders bevorzugt als Reinstoff oder als Lösung in einem bevorzugten organischen Lösungsmittel in Konzentrationen von 60-90 Gew.% und ganz besonders bevorzugt als Reinstoff oder als Lösung in einem bevorzugten Lösungsmittel in Konzentrationen von 60-85 Gew.% eingesetzt.

In Schritt (1a) wird bevorzugt ein organisches Lösungsmittel eingesetzt. Geeignete organische Lösungsmittel sind beispielsweise: aromatische oder aliphatische Halogenkohlenwasserstoffe, insbesondere aromatische oder aliphatische Chlorkohlenwasserstoffe, wie Tetrachlorethan, Dichlorpropan, Dichlormethan, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol und Trichlorbenzol; Ester, insbesondere Methylacetat, Ethylacetat, Propyl (n-und iso-)acetat oder Butylacetat; Ether, insbesondere Tetrahydrofuran (THF), 2-Methyl-THF, Cyclopentylmethylether, tert-Butylmethylether oder Diethylether; gegebenenfalls substituierte aliphatische, cycloaliphatische oder aromatische Kohlenwasserstoffe, insbesondere Pentan, Hexan, Heptan, Oktan, Nonan, Cyclohexan, Methylcyclohexan, Petrolether, Ligroin, Benzol, Toluol, Anisol, Xylol, Mesitylen oder Nitrobenzol; sowie Nitrile, insbesondere Acetonitril oder Propionitril oder Mischungen davon.

Bevorzugte Lösungsmittel in Schritt (1a) sind Ethylacetat, Propyl (n- und iso-)acetat, Butylacetat, 2-Methyl-THF, Cyclopentylmethylether, tert-Butylmethylether, Diethylether, Pentan, Hexan, Heptan, Oktan, Nonan, Cyclohexan, Methylcyclohexan, Petrolether, Ligroin, Benzol, Toluol, Anisol, Xylol, Mesitylen oder Nitrobenzol, Propionitril oder Mischungen davon. Besonders bevorzugte Lösungsmittel sind Ethylacetat, Isopropylacetat, tert-Butylmethylether, Cyclopentylmethylether, Methyl-THF oder Mischungen davon. Ganz besonders bevorzugt sind tert-Butylmethylether, Ethylacetat und Isopropylacetat oder Mischungen davon.

Die Lösungsmittel können in Kombination von zwei oder mehr in den Mischungen eingesetzt werden.

Besonders bevorzugt wird in Schritt (1a) ein organisches Lösungsmittel, das ausgewählt ist aus Ethylacetat, Isopropylacetat, tert-Butylmethylether, Cyclopentylmethylether, Methyl-THF oder Mischungen davon, eingesetzt, insbesondere zusammen mit Wasser.

Das Lösungsmittel oder Mischungen davon können mit Wasser gemischt werden. Dabei kann sich ein einphasiges oder ein zweiphasiges System ausbilden.

Vorzugsweise werden dabei 5 Volumina des Lösungsmittels oder der Mischungen davon mit 1 Volumen Wasser bis 1 Volumen des Lösungsmittel oder der Mischungen davon mit 5 Volumina Wasser gemischt.

Schritt (1a) wird bevorzugt in einem zweiphasigen System aus einem der oben genannten erfindungsgemäßen organischen Lösungsmittel oder Mischungen davon und Wasser durchgeführt. Dabei wird das organische Lösungsmittel oder Mischungen davon mit Wasser beispielsweise im Verhältnis von 5 Volumina : 1 Volumen bis 1 Volumen : 5 Volumina (Organisches Lösungsmittel oder Mischungen davon : Wasser), besonders bevorzugt in einem Verhälnis von 5 Volumina : 1 Volumen bis 1 Volumen : 2 Volumina, ganz besonders bevorzugt in einem Verhältnis von 2 Volumina : 1 Volumen bis 1 Volumen : 2 Volumina , am meisten bevorzugt in einem Verhältnis von 1 Volumen : 1 Volumen durchgeführt.

Bevorzugt wird Schritt (1a) in Anwesenheit eines Phasentransfer-Katalysators durchgeführt, insbesondere wenn Schritt (1a) in einem zweiphasigen System durchgeführt wird. Als Phasentransfer-Katalysator können verschiedene, dem Fachmann bekannte Verbindungen verwendet werden. Der Phasentransfer-Katalysator ist bevorzugt ausgewählt aus quartären Ammoniumsalzen (insbesondere Tetra-n-butylammonium-hydrogensulfat, -chlorid oder -bromid) und Tetraalkyl-phosphoniumsalzen (insbesondere Tri-n-butyl(tetradecyl)butylphosphoniumchlorid oder Trihexyltetradecylphosphoniumchlorid) oder Mischungen davon. Besonders bevorzugt ist der Phasentransfer-Katalysator ausgewählt aus Tetra-n-butylammonium-hydrogensulfat oder Tri-n-hexyltetradecylphosphoniumchlorid. Ganz besonders bevorzugt ist der Phasentransfer-Katalysator Tetra-n-butylammonium-hydrogensulfat.

Bevorzugt wird der Phasentransfer-Katalysator erfindungsgemäß in einem Anteil von 0,005 bis 0,06 Äquivalenten, besonders bevorzugt in einem Anteil von 0,01 bis 0,05 Äquivalenten, bezogen auf die gesamte eingesetzte Stoffmenge an Verbindung (II), eingesetzt. Der Katalysator wird dabei bevorzugt als Reinstoff eingesetzt.

Schritt (1a) wird bevorzugt in Anwesenheit eines Reduktionsmittels, beispielsweise Natrium- oder Kaliumdithionit, besonders bevorzugt Natriumdithionit, durchgeführt. Dabei werden erfindungsgemäß bevorzugt 0,9 bis 2,0 Äquivalente, besonders bevorzugt 1,0 bis 1,5 Äquivalente, ganz besonders bevorzugt 1,0 bis 1,2 Äquivalente, am meisten bevorzugt 1,1 Äquivalente bezogen auf die gesamte eingesetzte Stoffmenge an Verbindung (II), eingesetzt. Das Reduktionsmittel wird dabei bevorzugt als Reinstoff eingesetzt.

Schritt (1a) und/oder (1b) wird bevorzugt bei einer Temperatur im Bereich von -10 °C bis 80 °C, besonders bevorzugt im Bereich von 0 °C bis 60 °C, ganz besonders bevorzugt im Bereich von 5 °C bis 40 °C, am meisten bevorzugt im Bereich von 10 °C bis 25 °C durchgeführt.

Bevorzugt wird Schritt (1a) im Bereich des Normaldrucks (1013 hPa) durchgeführt. Schritt (1a) kann auch im Bereich von 300 hPa bis 5000 hPa oder von 500 hPa bis 2000 hPa oder von 1013 hPa ± 200 hPa durchgeführt werden.

Die Reaktionsdauer von Schritt (1a) liegt bevorzugt im Bereich von 3 bis 48 Stunden, besonders bevorzugt von 3 bis 24 Stunden, ganz besonders bevorzugt von 6 bis 24 Stunden.

Die Zugabe der Verbindungen R²-Y in Schritt (1a) erfolgt bevorzugt durch kontinuierliche Dosierung über einen Zeitraum von 0,5 bis 12 Stunden, besonders bevorzugt von 2 bis 10 Stunden, ganz besonders bevorzugt von 3 bis 6 Stunden.

Schritt (1a) wird bevorzugt unter pH-Kontrolle durchgeführt. Der pH-Wert der Reaktionslösung wird dabei bevorzugt in einem pH-Bereich von 3 bis 7, besonders bevorzugt in einem pH-Bereich von 4 bis 7 gehalten. Die pH-Kontrolle erfolgt bevorzugt sowohl während der Zugabe der Verbindungen R²-Y, als auch während der nachfolgenden Reaktion über die gesamte Reaktionszeit. Die pH-Kontrolle erfolgt bevorzugt durch Zugabe einer dem Fachmann allgemein bekannten, geeigneten Base, beispielsweise als Reinstoff oder wässrige Lösungen von (Erd-)Alkalicarbonaten, (Erd-)Alkalihydrogencarbonaten oder (Erd-)Alkalihydroxiden. Gegebenenfalls kann es vorteilhaft sein, den pH-Wert der Reaktionsmischung vor Beginn der Dosierung der Verbindungen R²-Y durch Zugabe einer dem Fachmann allgemein bekannten, geeigneten Säure einzustellen. Beispiele für Säuren sind Carbonsäuren, Mineralsäuren oder Sulfonsäuren. Beispiele für Carbonsäuren sind Essigsäure oder Propionsäure. Beispiele für Mineralsäuren sind Salzsäure oder Schwefelsäure. Ein Beispiel für eine Sulfonsäure ist Methansulfonsäure. Dabei wird auf einen bevorzugten pH-Wert, insbesondere einen pH-Wert von 4 bis 5, eingestellt.

Für die Umsetzung mit einer Verbindung der Formel R⁴SO₃H, wobei R⁴ wie oben definiert ist, in Schritt (1b) werden bevorzugt 0,9 bis 2,0 Äquivalente, besonders bevorzugt 0,9 bis 1,1 Äquivalente, ganz besonders bevorzugt 0,95 bis 1,0 Äquivalente, am meisten bevorzugt 1,0 Äquivalente bezogen auf die gesamte eingesetzte Stoffmenge der Verbindungen der Formel (II), der Verbindungen der Formel R⁴SO₃H eingesetzt. Diese Mengen werden vorzugsweise auch für eine Umsetzung mit einer aromatischen Carbonsäure R⁵CO₂H eingesetzt.

Werden die Verbindungen der Formel (IIa) in Schritt (1a) hergestellt, kann Schritt (1b) nach oder gleichzeitig mit Schritt (1a) erfolgen. Vorzugsweise erfolgt Schritt (1a) vor Schritt (1b). Die Zugabereihenfolge der Reagenzien in Schritt (1a) und/oder (1b) kann beliebig sein.

Es hat sich als vorteilhaft erwiesen, die Reaktionsmischung aus Schritt (1a) einer wässrigen Aufarbeitung zu unterziehen. Dabei wird vorzugsweise das Produkt aus Schritt (1a) in der organischen Phase gewonnen. Die organische Phase kann unmittelbar für die Umsetzung mit einer Verbindung der Formel R⁴SO₃H oder mit einer aromatischen Carbonsäure R⁵CO₂H in Schritt (1b) verwendet werden. Das Produkt aus Schritt (1a) kann aber auch nach Eindampfen der organischen Phase erneut in einem der für Schritt (1b) einsetzbaren Lösungsmitteln gelöst werden.

Dabei ist die Umsetzung in Schritt (1b) vorzugsweise eine Salzbildung zwischen den Verbindungen der Formel (IIa) und der Verbindung der Formel R⁴SO₃H oder der aromatischen Carbonsäure R⁵CO₂H. Insbesondere wenn die Verbindungen der Formel (IIa) in einem Schritt (1a) durch Umsetzung von Verbindungen der Formel (II) mit Verbindungen der Formel R²-Y, wobei Y für Iod oder Brom steht, gebildet werden, können neben den Verbindungen der Formel (IIa) auch Isomere davon gebildet werden, bei denen der Rest R² nicht in *para*-Position sondern in ortho-Position zur Aminogruppe ist, z.B. Verbindung A. Ferner können dabei Verbindungen mit zwei Resten R² entstehen, wobei einer der Reste in *para*-Position und der andere in ortho-Position zur Aminogruppe ist, z.B. Verbindung B.

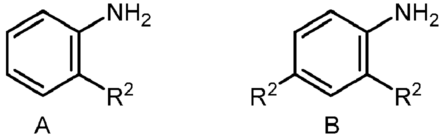

Die Salzbildung in Schritt (1b) erfolgt insbesondere in Anteilen, vorzugsweise quantitativ.

In Schritt (1b) wird bevorzugt ein organisches Lösungsmittel eingesetzt. Geeignete organische Lösungsmittel sind beispielsweise: aromatische oder aliphatische Halogenkohlenwasserstoffe, insbesondere aromatische oder aliphatische Chlorkohlenwasserstoffe, wie Tetrachlorethan, Dichlorpropan, Dichlormethan, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol und Trichlorbenzol; Ester, insbesondere Methylacetat, Ethylacetat, Propyl (n-und iso-)acetat oder Butylacetat; Ether, insbesondere Tetrahydrofuran (THF), 2-Methyl-THF, Cyclopentylmethylether, tert-Butylmethylether oder Diethylether; gegebenenfalls substituierte aliphatische, cycloaliphatische oder aromatische Kohlenwasserstoffe, insbesondere Pentan, Hexan, Heptan, Oktan, Nonan, Cyclohexan, Methylcyclohexan, Petrolether, Ligroin, Benzol, Toluol, Anisol, Xylol, Mesitylen oder Nitrobenzol; sowie Nitrile, insbesondere Acetonitril oder Propionitril oder Mischungen davon.

Bevorzugte Lösungsmittel sind Methylacetat, Ethylacetat, Propyl (n- und iso-)acetat, Butylacetat, Tetrahydrofuran (THF), 2-Methyl-THF, Cyclopentylmethylether, tert-Butylmethylether, Diethylether, Pentan, Hexan, Heptan, Oktan, Nonan, Cyclohexan, Methylcyclohexan, Petrolether, Ligroin, Benzol, Toluol, Anisol, Xylol, Mesitylen oder Nitrobenzol, Acetonitril, Propionitril oder Mischungen davon. Besonders bevorzugte Lösungsmittel sind Acetonitril, Methylacetat, Ethylacetat, Isopropylacetat, tert-Butylmethylether, Cyclopentylmethylether, THF oder Methyl-THF, Heptan oder Mischungen davon.

Die in Schritt (1b) gebildeten Salze sind vorzugsweise in der Reaktionsmischung von Schritt (1b) nicht oder nur wenig löslich. Die in Schritt (1b) gebildeten Salze fallen vorzugsweise als Feststoff in der Reaktionsmischung von Schritt (1b) an. Schritt (1b) umfasst somit vorzugsweise neben der Umsetzung mit der Verbindung der Formel R⁴SO₃H oder der aromatischen Carbonsäure R⁵CO₂H auch die Fällung der gebildeten Salze. Überraschend wurde gefunden, dass insbesondere Verbindungen der Formel (III) mit hoher Ausbeute und Reinheit auch dann ausfallen, wenn die Verbindungen der Formel (IIa) in einem Schritt (1a) durch Umsetzung von Verbindungen der Formel (II) mit Verbindungen der Formel R²-Y hergestellt wurden und zusammen mit Isomeren und zweifach substituierten Verbindungen, wie vorstehend beschrieben, vorliegen.

Die gebildeten Salze von Schritt (1b) können als amorpher Feststoff aus der Reaktionsmischung von Schritt (1b) ausfallen. Die gebildeten Salze von Schritt (1b) können in der Reaktionsmischung von Schritt (1b) kristallisieren. Dabei können die gebildeten Salze von Schritt (1b) auch als Gemische mit einem kristallinen Feststoffanteil und einem amorphen Feststoffanteil entstehen. Vorzugsweise läuft die Fällung und/oder Kristallisation quantitativ ab.

Die Verbindungen der Formel R⁴SO₃H oder der aromatischen Carbonsäure R⁵CO₂H werden in Schritt (1b) bevorzugt als Lösung in Konzentrationen von 5-80 Gew.%, besonders bevorzugt in Konzentrationen von 10-50 Gew.% und ganz besonders bevorzugt in Konzentrationen von 15-40 Gew.% eingesetzt. Bevorzugte Lösungsmittel für Lösungen der Verbindungen der Formel R⁴SO₃H oder der aromatischen Carbonsäure R⁵CO₂H sind Ether, Ester oder Alkohole oder Mischungen davon. Besonders bevorzugt sind Cyclopentylmethylether, tert Butylmethylether, Diethylether, Ethylacetat, oder Mischungen davon. Ganz besonders bevorzugt ist tert Butylmethylether.

Die Umsetzung in Schritt (1b) kann in verschiedenen Lösungsmitteln erfolgen. Als Lösungsmittel für die Umsetzung mit einer Verbindung der Formel R⁴SO₃H in Schritt (1b) kommen insbesondere die vorstehend genannten Lösungsmittel für die Verbindungen der Formel R⁴SO₃H oder für die aromatischen Carbonsäure R⁵CO₂H in Betracht. Wurden die Verbindungen der Formel (IIa) durch Umsetzung von Verbindungen der Formel (II) mit Verbindungen der Formel R²-Y in einem Schritt (1a) erhalten, so kann insbesondere das Lösungsmittel der Umsetzung aus Schritt (1a) oder das Lösungsmittel der organischen Phase nach wässriger Aufarbeitung zum Einsatz kommen. Ebenfalls denkbar ist eine Mischung des Lösungsmittels der organischen Phase nach wässriger Aufarbeitung mit dem Lösungsmittel für die Verbindungen der Formel R⁴SO₃H oder für die aromatischen Carbonsäure R⁵CO₂H.

Dementsprechend sind bevorzugte Lösungsmittel für die Umsetzung mit einer Verbindung der Formel R⁴SO₃H oder der aromatischen Carbonsäure R⁵CO₂H in Schritt (1b) Ether oder Ester oder Mischungen davon. Besonders bevorzugt sind, Methylacetat, Ethylacetat, Isopropylacetat, tert-Butylmethylether, Cyclopentylmethylether, THF und Methyl-THF oder Mischungen davon. Ganz besonders bevorzugt sind Mischungen von tert Butylmethylether und oder Ethylacetat.

Die Lösungsmittel können in Kombination von zwei oder mehr Lösungsmitteln in den Mischungen eingesetzt werden.

Die Umsetzung mit einer Verbindung der Formel R⁴SO₃H in Schritt (1b) kann in einer Mischung aus den bevorzugten organischen Lösungsmittel erfolgen, wobei die organischen Lösungsmittel beispielsweise in einem Verhältnis von 5 Volumina : 1 Volumen bis 1 Volumen : 5 Volumina, besonders bevorzugt in einem Verhältnis von 4 Volumina : 1 Volumen bis 1 Volumen : 4 Volumina, ganz besonders bevorzugt in einem Verhältnis von 3 Volumina : 1 Volumen bis 1 Volumen : 3 Volumina gemischt werden.

Für die Fällung in Schritt (1b) kann der Reaktionsmischung der Umsetzung von Schritt (1b) ein weiteres Lösungsmittel, insbesondere als Antisolvens, zugegeben werden. Ein Antisolvens kann die Kristallisation verbessern.

Als Antisolvens können in der Fällung von Schritt (1b) zur Verbesserung der Kristallisation insbesondere substituierte aliphatische, cycloaliphatische oder aromatische Kohlenwasserstoffe zugesetzt werden, beispielsweise Hexan, Heptan, Octan, Cyclopentan, Cyclohexan, Methylcyclohexan, Cycloheptan, Toluol, oder Xylol. Bevorzugt werden als Antisolvens Hexan, Heptan, Methylcylohexan oder Cyclohexan, besonders bevorzugt Heptan oder Methylcyclohexan, zugesetzt. Die Fällung in Schritt (1b) kann somit beispielsweise in einer Mischung von Ethylacetat, tert Butylmethylether und Heptan durchgeführt werden.

Die Vereinigung von Verbindungen der Formel (IIa) mit der Verbindung der Formel R⁴SO₃H oder der aromatischen Carbonsäure R⁵CO₂H kann für deren Umsetzung auf unterschiedliche Art und Weise bewerkstelligt werden. Wie vortsehend beschrieben, werden zweckmäßigerweise eine Lösung enthaltend die Verbindungen der Formel (IIa) und eine Lösung der Verbindung der Formel R⁴SO₃H oder der aromatischen Carbonsäure R⁵CO₂H eingesetzt. Die Lösung enthaltend Verbindungen der Formel (IIa) kann der Lösung der Verbindung der Formel R⁴SO₃H oder der aromatischen Carbonsäure R⁵CO₂H zugegeben werden oder umgekehrt. Die Zugabe erfolgt dabei langsam, vorzugsweise über einen Zeitraum von 0,5 bis 4 Stunden, besonders bevorzugt 1 bis 3 Stunden. Dadurch kann entstehende Reaktionswärme gut abgeführt werden. Die Zugabe kann kontinuierlich erfolgen. Die Zugabe kann auch tropfenweise erfolgen.

Werden die in Schritt (1b) gebildeten Salze gefällt und wird der Reaktionsmischung der Umsetzung hierfür ein Antisolvens zugegeben, so wird das Antisolvens vorzugsweise langsam zugegeben, bevorzugt über einen Zeitraum von 0,5 bis 4 Stunden, besonders bevorzugt 1 bis 3 Stunden. Durch eine langsame Zugabe kann eine zu rasche und gegebenenfalls unsaubere Fällung bzw. Kristallisation vermieden werden.

Nach Beendigung der Vereinigung von Verbindungen der Formel (IIa) mit der Verbindung der Formel R⁴SO₃H oder nach Zugabe eines Antisolvens zu der resultierenden Reaktionsmischung in Schritt (1b) wird vorzugsweise für einen Zeitraum von 1 bis 48 Stunden, besonders bevorzugt 2 bis 24 Stunden gerührt. Dadurch kann die Fällung bzw. Kristallisation vervollständigt werden. Die gesamte Dauer von Schritt (1b) liegt bevorzugt im Bereich von 2 bis 72 Stunden, besonders bevorzugt von 3 bis 30 Stunden, ganz besonders bevorzugt von 6 bis 24 Stunden.

Die Umsetzung mit einer Verbindung der Formel R⁴SO₃H in Schritt (1b) wird bevorzugt bei einer Temperatur im Bereich von -10 °C bis 75 °C, weiter bevorzugt -10 °C bis 50 °C, besonders bevorzugt im Bereich von 5 °C bis 25 °C, ganz besonders bevorzugt im Bereich von 10 °C bis 20 °C durchgeführt.

Die Fällung in Schritt (1b) wird bevorzugt bei einer Temperatur von -20 °C bis 40 °C, besonders bevorzugt -10 °C bis 30 °C, ganz besonders bevorzugt von 0 °C bis 20 °C durchgeführt. Tiefere Temperaturen begünstigen die Fällung. Wird die Fällung bei tiefen Temperaturen durchgeführt, kann ggfs. auf die Zugabe eines Antisolvens verzichtet werden.

Vor der Fällung kann auch ein Teil des Lösungsmittels für die Umsetzung entfernt werden. Wird ein Teil des Lösungsmittel für die Umsetzung vor der Fällung entfernt, kann auf die Zugabe eines Antisolvens verzichtet werden.

Bevorzugt wird Schritt (1b) im Bereich des Normaldrucks (1013 hPa) durchgeführt. Schritt (1b) kann auch im Bereich von 300 hPa bis 5000 hPa oder von 500 hPa bis 2000 hPa oder von 1013 hPa ± 200 hPa durchgeführt werden.

Wie vorstehend beschrieben, können insbesondere Verbindungen der Formel (III) mit hoher Ausbeute und Reinheit auch dann ausfallen, wenn die Verbindungen der Formel (IIa) in einer Mischung mit anderen Verbindungen, insbesondere Isomeren von Verbindungen der Formel (IIa) oder zweifach substituierten Verbindungen vorliegen.

Dabei wurde überraschend gefunden, dass insbesondere die Umsetzung mit einer Verbindung der Formel R⁴SO₃H in Schritt (1b) der Umsetzung mit anorganischen Säuren, z.B. Mineralsäuren wie Salzsäure oder Schwefelsäure, oder nicht-aromatischen Carbonsäuren, z.B. Essigsäure, oder Perfluorcarbonsäuren, z.B. Trifluoressigsäure, in Schritt (1b) überlegen ist, da mit ersterer insbesondere die Verbindungen der Formel (III) mit höheren Ausbeuten und mit höherer Reinheit erhalten werden können. Auch die Umsetzung mit einer aromatischen Carbonsäure R⁵CO₂H ergibt die Verbindungen der Formel (IIIa) mit guter Ausbeute und guter Reinheit.

Für die Umsetzung mit einer Verbindung der Formel R⁴SO₃H in Schritt (1b) können Verbindungen der Formel (III) mit einem hohen Anteil an Feststoff erhalten werden. Die Umsetzung mit einer Verbindung der Formel R⁴SO₃H in Schritt (1b) kann somit Verbindungen der Formel (III) mit hoher Ausbeute, hoher Selektivität und hoher Reinheit ergeben. Die Verbindungen der Formel (III) können mit hoher Produktqualität auch in großtechnischem Maßstab auf einfache Weise isoliert werden. Die Bildung unerwünschter Nebenprodukte wird optimiert. Aufwendige und kostenintensive Aufarbeitungsschritte sowie kostenintensive Materialien zur Aufarbeitung entfallen.

Die Verbindungen der Formel (III) sind somit zum Einsatz in einem GMP-regulierten Umfeld geeignet. Weiterhin ist vorteilhaft, dass Verbindungen der Formel R⁴SO₃H kostengünstige Reagenzien sind, die auch in großem Maßstab bereit gestellt werden können, und deren Verwendung ein kosteneffektives Verfahren für die Herstellung von Verbindungen der Formel (I) ermöglicht.

### Schritt (2), Halogenierung:

Erfindungsgemäß werden die Verbindungen der Formel (III) oder (IIIa), vorzugsweise der Formel (III) mit einem Halogenierungsmittel oder Mischungen davon in Schritt (2) zu Verbindungen der Formel (I) umgesetzt.

Der Begriff "Halogenierungsmittel" wird bevorzugt für Chlorierungs-, Bromierungs-, Fluorierungs- und Iodierungsmittel verwendet.

Im nachfolgenden Abschnitt der Beschreibung betreffend Schritt (2), steht der Begriff *Halogen* bevorzugt für Chlor oder Brom.

In Schritt (2) wird ein Halogenierungsmittel eingesetzt. Geeignete Halogenierungsmittel sind die dem Fachmann allgemein bekannten Halogenierungsmittel. Geeignete Halogenierungsmittel können beispielsweise Chlor, Brom, ein anorganisches Chlor- oder Brom-haltiges Salz, oder ein organisches Chlor- oder Brom-haltiges Molekül, in dem die Bindung eines organischen Restes zum Halogen-Atom polarisiert ist, so dass das Chlor oder Brom-Atom Träger einer partiell positiven Ladung ist, wie zum Beispiel *N*-Halogensuccinimide, 1,3-Dihalogen-5-5-dimethylhydantoine oder Halogencyanursäuren (organische halogenierende Verbindungen) oder Mischungen davon sein.

Bevorzugte Halogenierungsmittel sind dabei Chlor, Brom oder organische Halogenierungsmittel, welche besonders bevorzugt ausgewählt sind aus *N*-Chlorsuccinimid (NCS), *N*-Bromsuccinimid (NBS), 1,3-Dichlor-5-5-dimethylhydantoin (DCDMH), 1,3-Dibrom-5-5-dimethylhydantoin (DBDMH), 1,3,5-Trichlor-1,3,5-triazin-2,4,6-trion (TCCA), 1,3,5-Tribrom-1,3,5-triazin-2,4,6-trion oder 1,3-Dibrom-1,3,5-triazin-2,4,6-trion. Ganz besonders bevorzugt sind die halogenierenden Verbindungen ausgewählt aus Chlor, Brom, *N*-Chlorsuccinimid (NCS) oder *N*-Bromsuccinimid (NBS), 1,3-Dichlor-5-5-dimethylhydantoin (DCDMH), 1,3-Dibrom-5-5-dimethylhydantoin (DBDMH), 1,3,5-Trichlor-1,3,5-triazin-2,4,6-trion (TCCA), insbesondere bevorzugt sind dabei Chlor, Brom, *N*-Chlorsuccinimid (NCS) oder *N*-Bromsuccinimid (NBS).

Die Halogenierungsmittel können alleine oder in Kombination von zwei oder mehr in den Mischungen eingesetzt werden, solange die eingesetzten Verbindungen dasselbe Halogen tragen.

Das Halogenierungsmittel kann erfindungsgemäß in einem Anteil von 1,0 bis 3,0 Äquivalente (*Mono-Halogenverbindungen*) bzw. von 0,5 bis 1,5 Äquivalente (*Di-Halogenverbindungen*) bzw. von 0,3 bis 1,0 Äquivalente (*Tri-Halogenverbindungen*) und bevorzugt von 1,0 bis 2,5 Äquivalente (*Mono-Halogenverbindungen*) bzw. von 0,5 bis 0,8 Äquivalente (*Di-Halogenverbindungen*) bzw. von 0,33 bis 0,75 Äquivalente (*Tri-Halogenverbindungen*), bezogen auf die gesamte eingesetzte Stoffmenge an Verbindung (III) oder (IIIa), eingesetzt werden. Chlor oder Brom werden bevorzugt bis zum vollständigen Umsatz durch die Lösung geleitet.

Gegebenenfalls kann ein Überschuss des Halogenierungsmittels nach mittels HPLC^{a} festgestelltem vollständigem Umsatz durch die Zugabe eines dem Fachmann allgemein bekannten Reduktionsmittels neutralisiert werden. Geeignete Reduktionsmittel können (Erd-)Alkalisulfite, (Erd-)Alkalidithionite oder (Erd-)Alkalithiosulfate sein. Die Reduktionsmittel können dabei bevorzugt als Reinstoff oder als wässrige, beispielsweise als gesättigte wässrige Lösung, eingesetzt werden.

Das Halogenierungsmittel kann erfindungsgemäß als Reinstoff in Form von Feststoff, Gas, Flüssigkeit, Suspension oder Lösung in einem geeigneten, unter den Reaktionsbedingungen inerten organischen Lösungsmittel eingesetzt werden. Das Halogenierungsmittel liegt vorzugsweise in dem für die Reaktion ausgewählten Lösungsmittel vor. Dabei wird das Halogenierungsmittel bevorzugt in einer Konzentration von 5-95 Gew.%, besonders bevorzugt in einer Konzentration von 10-80 Gew.% eingesetzt. Geeignete organische Lösungsmittel sind insbesondere die für Schritt (2) genannten bevorzugten Lösungsmittel.

Besondere Katalysatoren sind für Schritt (2) nicht nötig. Unter Umständen kann es vorteilhaft sein, Säuren in katalytischen Mengen zur Aktivierung zu nutzen. Säuren in katalytischer Menge können insbesondere zusätzlich zu der Säuremenge eingesetzt werden, die durch die Verbindung der Formel R⁴SO₃H in der Verbindung der Formel (III) oder durch die aromatische Carbonsäure R⁵CO₂H in der Verbindung der Formel (IIIa) vorhanden ist. Dies ist jedoch bei den hier beanspruchten Reaktionen nicht zwingend nötig. Insbesondere können Katalysatoren vorteilhaft bei der Verwendung von organischen Chlorierungsmitteln wie beispielsweise *N*-Chlorsuccinimid (NCS) und 1,3-Dichlor-5-5-dimethylhydantoin (DCDMH) sein. Als Katalysator geeignete Säuren können dabei bevorzugt ausgewählt sein aus den dem Fachmann geläufigen Mineralsäuren, Sulfonsäuren, Carbonsäuren oder Lewis-Säuren. Mineralsäuren sind beispielsweise Schwefelsäure, Salzsäure und Fluorwasserstoffsäure. Als Sulfonsäuren können beispielsweise Methansulfonsäure, Trifluormethansulfonsäure und 4-Toluolsulfonsäure verwendet werden. Beispiele für Carbonsäuren sind Trifluoressigsäure und Trichloressigsäure. Als Lewis-Säuren können beispielsweise Eisen(III)- und Scandium(III)trifluormethansulfonat eingesetzt werden.

Die Reaktion wird bevorzugt in einem Temperaturbereich von -78 bis 200 °C, besonders bevorzugt bei Temperaturen von -20 bis 100 °C, ganz besonders bevorzugt von 0 °C bis 50 °C und am meisten bevorzugt von 0 °C bis 25 °C durchgeführt.

Die Reaktion kann unter erhöhtem als auch vermindertem Druck ausgeführt werden. Bevorzugt wird sie unter Normaldruck durchgeführt. Sie kann aber auch im Bereich von 1013 hPa±300 hPa oder im Bereich von 1013 hPa±100 hPa oder im Bereich von 1013 hPa±50 hPa durchgeführt werden.

Schritt (2) wird bevorzugt in einem organischen Lösungsmittel durchgeführt. Als Verdünnungs- bzw. Lösungsmittel zur Durchführung von Schritt (2) kommen grundsätzlich alle unter den spezifischen Reaktionsbedingungen inerten organischen Lösungsmittel in Frage.

Als Beispiele für geeignete organische Lösungsmittel sind zu nennen: aromatische oder aliphatische Halogenkohlenwasserstoffe, insbesondere aromatische oder aliphatische Chlorkohlenwasserstoffe, wie Tetrachlorethan, Dichlorpropan, Dichlormethan, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol oder Trichlorbenzol; Nitrile, insbesondere Acetonitril, Propionitril, Butyronitril, Isobutyronitril, Benzonitril oder m-Chlorbenzonitril; gegebenenfalls substituierte aliphatische, cycloaliphatische oder aromatische Kohlenwasserstoffe, insbesondere Pentan, Hexan, Heptan, Oktan, Nonan, Cyclohexan, Methylcyclohexan, Petrolether, Ligroin oder Nitrobenzol; Ester, insbesondere Methyl-, Ethyl-, Isopropyl-, Butyl-, Isobutylacetat, Dimethyl-, Dibutyl- oder Ethylencarbonat; aliphatische oder cycloaliphatische Ether, insbesondere 1,2-Dimethoxyethan (DME), Diglyme, Tetrahydrofuran (THF), 2-Methyl-THF, 1,4-Dioxan, tert-Butylmethylether, Cyclopentylmethylether, Aceton, Methylethylketon, Methyl-tert-butylketon oder Methylisobutylketon.

Bevorzugte Verdünnungs- bzw. Lösungsmittel sind Acetonitril, Propionitril, Butyronitril, Isobutyronitril, Benzonitril, Alkane wie Pentan, Hexan, Heptan, Oktan, Nonan, Cyclohexan, Methylcyclohexan, Petrolether, Ligroin, Nitrobenzol, Methyl-, Ethyl-, Isopropyl-, Butyl-, Isobutylacetat, Dimethyl-, Dibutyl-oder Ethylencarbonat, 1,2-Dimethoxyethan (DME), Diglyme, Tetrahydrofuran (THF), 2-Methyl-THF, 1,4-Dioxan, tert-Butylmethylether, Cyclopentylmethylether, Essigsäure, *n*-Propansäure, *n*-Butansäure oder Mischungen davon.

Besonders bevorzugte Verdünnungs- bzw. Lösungsmittel sind aromatische oder aliphatische halogenierte Kohlenwasserstoffe, insbesondere Chlorbenzol, Dichlorbenzol, Dichlormethan, Chloroform, 1,2-Dichlorethan oder Tetrachlorkohlenstoff; Ester, insbesondere Ethylacetat, Isopropylacetat und Butylacetat; Ether, insbesondere Tetrahydrofuran (THF), 2-Methyl-THF, tert-Butylmethylether oder Cyclopentylmethylether; Nitrile, insbesondere Acetonitril oder Propionitril, Aceton, Methylethylketon, Methyl-tert-butylketon, Methylisobutylketon, Alkane wie Hexan, Heptan, Methylcylohexan oder Cyclohexan oder Mischungen davon.

In einer ganz besonders bevorzugten Ausführungsform ist das Lösungsmittel ausgewählt aus Acetonitril, Ethylacetat, Isopropylacetat, tert-Butylmethylether, Cyclopentylmethylether, Methyl-THF, insbesondere 2-Methyl-THF, Aceton, Methylethylketon, Methyl-tert-butylketon, Methylisobutylketon, Alkane wie Hexan, Heptan, Methylcyclohexan oder Cyclohexan oder Mischungen davon. Ganz besonders bevorzugt sind Acetonitril, tert-Butylmethylether, Ethylacetat und Isopropylacetat oder Mischungen davon.

Die Lösungsmittel können allein oder in Kombination von zwei oder mehr in den Mischungen eingesetzt werden.

Die Dauer der Halogenierung der Verbindungen der Formel (III) oder (IIIa) liegt bevorzugt im Bereich von 0,5 Stunden bis 80 Stunden, besonders bevorzugt im Bereich von 1 Stunde bis 72 Stunden, ganz besonders bevorzugt 1 Stunde bis 24 Stunden. Eine längere Reaktionsdauer ist möglich, jedoch aus wirtschaftlicher Sicht nicht sinnvoll.

Die Zugabereihenfolge der Reaktanden ist beliebig. Das Halogenierungsmittel kann vorgelegt und die übrigen Reaktanden hinzugegeben werden. Das Halogenierungsmittel kann auch zu den übrigen Reaktanden zugegeben werden. Bevorzugt wird das Halogenierungsmittel zugegeben. Das Halogenierungsmittel kann in einer Portion oder durch Dosierung über einen längeren Zeitraum zugegeben werden. Unter Umständen kann es auch von Vorteil sein, eine Lösung der Verbindung (III) oder (IIIa) in einem für Schritt (2) genannten Lösungsmittel zu einer Lösung oder Suspension des Halogenierungsmittels in einem für Schritt (2) bevorzugten Lösungsmittel zuzugeben. Es kann auch zudosiert werden, vorzugsweise langsam hinzu dosiert werden. Die Dauer der Dosierung kann dabei in einem bevorzugten Bereich von 0,5 bis 6 Stunden, besonders bevorzugt von 1 bis 4 Stunden liegen. Längere Dosierzeiten sind aus technischer Sicht auch möglich, sind jedoch aus wirtschaftlicher Sicht nicht sinnvoll.

Die Zugabe oder Dosierung erfolgt bevorzugt in einem Temperaturbereich von -78 bis 200 °C, besonders bevorzugt bei Temperaturen von -20 bis 100 °C, ganz besonders bevorzugt von 0 °C bis 50 °C und am meisten bevorzugt von 0 °C bis 25 °C. In einer vorteilhaften Ausgestaltung entspricht die Temperatur, bei der die Zugabe oder Dosierung erfolgt, der Reaktionstemperatur.

In einer besonders vorteilhaften Ausgestaltung der Erfindung wird in den Schritten (1a) und/oder (1b) und Schritt (2) dasselbe organische Lösungsmittel eingesetzt.

Im Rahmen dieser Ausgestaltung der Erfindung ist das Lösungsmittel in den Schritten (1a), (1b) und (2) bevorzugt ausgewählt aus Ester, Ether oder Nitrile oder Mischungen davon. Besonders bevorzugt ist das Lösungsmittel ausgewählt aus Ethylacetat, Isopropylacetat, tert-Butylmethylether, Cyclopentylmethylether, THF, Methyl-THF oder Acetonitril oder Mischungen davon. Ganz besonders bevorzugt sind Acetonitril, tert-Butylmethylether, Ethylacetat oder Isopropylacetat oder Mischungen davon.

Die genannten Lösungsmittel können allein oder in Kombination von zwei oder mehr in den Mischungen eingesetzt werden.

Das erfindungsgemäße Verfahren zeichnet sich dadurch aus, dass die Verbindungen der Formel (III) oder (lila) auch aus Mischungen verschiedener Verbindungen, beispielsweise dem Reaktionsgemisch von Schritt (1a), in Form eines Feststoffes, vorzugsweise in Form eines hochreinen Feststoffes, und in hoher Ausbeute isoliert werden können. Die Verbindungen der Formel (III) oder (IIIa) werden in Schritt (2) bevorzugt unter milden Bedingungen im Wesentlichen zersetzungsfrei umgesetzt. Die Verbindungen der Formel (III) oder (IIIa) aus Schritt (1b) können unmittelbar in Salzform, vorzugsweise in fester, in gelöster oder suspendierter Form, in Schritt (2) eingesetzt werden. Für die Durchführung von Schritt (2) ist es nicht erforderlich, die Verbindungen der Formel (III) oder (IIIa) als freie Basen einzusetzen. Die Verbindungen der Formel (III) oder (IIIa) können auch als freie Basen, vorzugsweise in fester, in gelöster oder suspendierter Form in Schritt (2) eingesetzt werden. Dabei kann der Feststoff in einem für Schritt (1a), (1b) und/oder Schritt (2) geeigneten Lösungsmittel gelöst oder suspendiert werden.

Der Begriff "Isolieren" im Sinne der vorliegenden Erfindung bedeutet eine vollständige Abtrennung der Verbindungen der Formel (III) oder (IIIa) oder der Verbindungen der Formel (I) aus dem Reaktionsgemisch, also beispielweise von allen Lösungsmitteln und Salzen durch dem Fachmann allgemein bekannte Trennverfahren. Vorzugsweise werden die Verbindungen der Formel (III) oder (IIIa) als Feststoff durch Filtrieren, Zentrifugieren oder Dekantieren abgetrennt. Vorzugsweise werden die Verbindungen der Formel (I) als Feststoff durch Filtrieren, Zentrifugieren oder Dekantieren abgetrennt.

Bei dem erfindungsgemäßen Verfahren können während der Reaktionsfolge Reaktionsvolumina in Form von Gasen, Feststoffen, Flüssigkeiten, Lösungen oder Suspensionen zugegeben werden. Halogenierungsmittel können in gasförmiger, flüssiger, fester, gelöster oder suspendierter Form zugegeben werden. Für Schritt (1a) und/oder (1b) geeignete Lösungsmittel können auch in Schritt (2) zugegeben werden. Insbesondere ist eine Zugabe von Säuren oder Basen möglich. Das teilweise oder vollständige Entfernen von wässrigen Bestandteilen der Reaktionsmischung in den Reaktionsschritten (1a) und (2) ist möglich.

Als aktives Entfernen des organischen Lösungsmittels wird im Allgemeinen das Entfernen des organischen Lösungsmittels mittels Distillation, gegebenenfalls unter thermischer Einwirkung auf das Reaktionsgemisch, unter Normal- oder vermindertem Druck verstanden.

Bevorzugt besteht das erfindungsgemäße Verfahren aus den Schritten (1a), (1b) und (2).

Gegebenenfalls können Schritt (1a), (1b) und/oder Schritt (2) auch mehrfach, beispielsweise zwei- oder dreimal, in dem gleichen Reaktionsgefäß ohne weitere Aufarbeitung ausgeführt werden. Die Reaktionsmischung aus Schritt (1a) kann beispielsweise nach vollständigem Umsatz gemäß HPLC^{a} erneut mit der Verbindung der Formel (II) sowie vorzugsweise einem erfindungsgemäßen Reduktionsmittel versetzt und durch Dosierung einer Verbindung R²-Y, vorzugsweise unter pH-Kontrolle, zu einer Verbindungen des Schritts (1a) umgesetzt werden. Dieser Vorgang kann erneut wiederholt oder die Reaktionsmischung erfindungsgemäß weiter behandelt werden. Die Reaktionsmischung aus Schritt (2) kann analog nach vollständigem Umsatz gemäß HPLC^{a} erneut mit der Verbindung der Formel (III) oder (lila) versetzt und anschließend durch Zugabe eines erfindungsgemäßen Halogenierungsmittels weiter zu Verbindungen der Formel (I) umgesetzt werden.

Die Aufarbeitung und Isolierung der Verbindungen (I) kann, vorzugsweise nach vollständiger Reaktion, erfolgen, z. B. durch Einengung oder Entfernung des Lösungsmittels, Waschen mit Wasser und Extraktion mit einem geeigneten organischen Lösungsmittel und Separation der organischen Phase sowie Fällen und/oder Kristallisation aus der organischen Phase, gegebenenfalls unter teilweiser Entfernung des Lösungsmittels unter vermindertem Druck und/oder unter Zugabe eines weiteren geeigneten Lösungsmittels. Gegebenenfalls kann nach Separation der organischen Phase das Lösungsmittel vollständig unter vermindertem Druck entfernt werden und der Rückstand weiterhin einer Vakuumdestillation bei 0,05-1 mbar mit einer Spaltrohrkolonne sowie einer Kristallisation in einem dem Fachmann allgemein bekannten Lösungsmittel unterzogen werden.

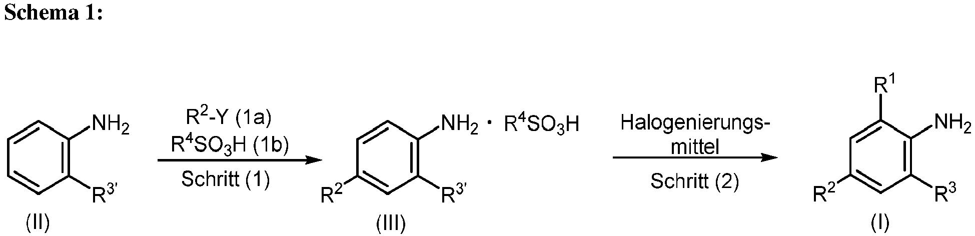

Schema 1 gibt eine schematische Gesamtdarstellung einer bevorzugten Ausführungsform des erfindungsgemäßen Verfahrens unter Verwendung einer Verbindung der Formel R⁴SO₃H und mit den Schritten (1) und (2) wieder, wobei Schritt (1) die Schritte (1a) und (1b) umfasst. Reaktionsbedingungen und Reaktanden werden dabei gemäß den oben beschriebenen erfindungsgemäßen und bevorzugten Ausgestaltungen ausgewählt. Alle Variablen in den Formeln (I), (II), (III), R²-Y und R⁴SO₃H sind wie oben beschrieben definiert.

Eine bevorzugte Ausführungsform des erfindungsgemäßen Verfahrens ist die folgende:
Die Verbindungen der Formel (II) werden in einem Gemisch eines organischen Lösungsmittels, z. B. Ethylacetat, und Wasser vorgelegt und nach Zugabe eines erfindungsgemäßen Phasentransferkatalysators, z.B. Tetra-n-butylammoniumhydrogensulfat oder Tri-n-hexyl(tetradecyl)phosphoniumchlorid, sowie eines erfindungsgemäßen Reduktionsmittels, z. B. Natriumdithionit, bei bevorzugt -5 °C bis 40 °C, besonders bevorzugt 10 °C bis 25 °C über bevorzugt 3 h bis 24 h, besonders bevorzugt 6 h bis 24 h, mit einer erfindungsgemäßen Verbindung der Formel R²-Y, z. B. Heptafluoro-2-iod-propan versetzt. Der pH-Wert der Reaktionsmischung wird dabei während der gesamten Reaktionsdauer bevorzugt durch Zugabe einer geeigneten Base, als Feststoff oder als wässrige Lösung, z.B. 40 Gew.% wässrige Kaliumcarbonat-Lösung, in einem Bereich von 3 bis 7 gehalten. Nach bevorzugt 6 h bis 24 h wird die wässrige Phase abgetrennt, die organische Phase gegebenenfalls mit wässriger Natriumchlorid-Lösung, z. B. 20 Gew. %, und/oder wässriger Salzsäure, z. B. 5 Gew. % oder 2,5 Gew.%, und/oder einer wässrigen Natriumchlorid-Lösung, z. B. 10 Gew. %, gewaschen. Somit werden die Produkte aus Schritt (1a) als Lösung in dem organischen Lösungsmittel, z. B. Ethylacetat, erhalten.

Zu einer Lösung aus einer Verbindung der Formel R⁴SO₃H, z. B. Methansulfonsäure, 4-Toluolsulfonsäure oder Benzensulfonsäure, in einem geeigneten organischen Lösungsmittel, z. B. tert Butylmethylether, wird bevorzugt langsam, besonders bevorzugt durch eine kontinuierliche Dosierung über einen bestimmten Zeitraum die Verbindungen von Schritt (1a) als Lösung in dem organischen Lösungsmittel, z. B. Ethylacetat, bei bevorzugt 15 °C bis 25 °C, besonders bevorzugt 20 °C, gegeben, gefolgt durch kontinuierliche Zugabe über einen bestimmten Zeitraum eines weiteren organischen Lösungsmittels, z.B. Heptan, bei bevorzugt 15 °C bis 25 °C, besonders bevorzugt 20 °C. Die Reaktionsmischung wird bevorzugt für 3 h bis 24 h, besonders bevorzugt für 6 h bis 24 h bei einer Temperatur in einem Bereich von bevorzugt 5 °C bis 25 °C, besonders bevorzugt von 15 °C bis 25 °C gerührt und anschließend bevorzugt für 1 h bis 3 h, besonders bevorzugt 1,5 h bis 2,5 h, bei bevorzugt 5 °C bis 15 °C, besonders bevorzugt 10 °C, gerührt. Die Verbindungen der Formel (III) fallen als Feststoff in der Reaktionsmischung an und werden anschließend isoliert, bevorzugt durch Filtration. Vorzugsweise wird der Feststoff in einem geeigneten organischen Lösungsmittel, z. B. tert Butylmethylether, bei bevorzugt 15 °C bis 25 °C, besonders bevorzugt 20 °C, suspendiert, erneut isoliert, bevorzugt durch Filtration, und getrocknet.

Die Verbindungen der Formel (III) werden bevorzugt bei -20 °C bis 100 °C, besonders bevorzugt bei 0 °C bis 25 °C, mit einem Halogenierungsmittel, z. B. als Gas, Feststoff, Flüssigkeit, Suspension oder Lösung in einem erfindungsgemäßen organischen Lösungsmittel, z. B. Ethylacetat, über bevorzugt 1 h bis 65 h versetzt. Nach vollständigem Umsatz (HPLC^{a}) wird gegebenenfalls vorhandenes überschüssiges Halogenierungsmittel durch die Zugabe eines Reduktionsmittels, beispielsweise als Reinstoff oder wässrige Lösung, neutralisiert. Die Reaktionsmischung wird gegebenenfalls ein oder mehrfach mit einer alkalischen wässrigen Lösung, z. B. Natriumhydrogencarbonat und/oder einer wässrigen Natriumchloridlösung, z. B. 15 Gew. %, bei bevorzugt 0 °C bis 25 °C gewaschen. Vorzugsweise werden die Waschschritte bei einer Temperatur, die der jeweiligen Reaktionstemperatur entspricht, durchgeführt. Das Lösungsmittel der organischen Phase wird unter vermindertem Druck teilweise entfernt. Vorzugsweise wird die reduzierte organische Phase mit einem weiteren geeigneten Lösungsmittel, z. B. Heptan, versetzt und bevorzugt für 0,5 h bis 3 h, besonders bevorzugt 1 h gerührt und anschließend als Feststoff angefallene Nebenprodukte des Halogenierungsmittels isoliert, bevorzugt durch Filtrieren. Nach Abtrennung des Feststoffs wird die verbleibende organische Phase langsam mit einem oder mehreren geeigneten weiteren Lösungsmittel, z. B. Methanol und Wasser, versetzt und die als Feststoff angefallenen Verbindungen der Formel (I) isoliert, bevorzugt durch Filtrieren. (Schritt (1) und (2)).

In einer weiteren vorteilhaften Ausführungsform werden die Verbindungen der Formel (II) in einem Gemisch eines organischen Lösungsmittels, z. B. Ethylacetat, und Wasser vorgelegt und nach Zugabe eines erfindungsgemäßen Phasentransferkatalysators, z. B. Tetra-n-butylammoniumhydrogensulfat oder Tri-n-hexyl(tetradecyl)phosphoniumchlorid, sowie eines erfindungsgemäßen Reduktionsmittels, z. B. Natriumdithionit, bei bevorzugt -5 °C bis 40 °C, besonders bevorzugt 10 °C bis 25 °C über bevorzugt 3 h bis 24 h, besonders bevorzugt 6 h bis 24 h, mit einer erfindungsgemäßen Verbindung der Formel R²-Y, z.B. Heptafluoro-2-iod-propan versetzt. Der pH-Wert der Reaktionsmischung wird dabei während der gesamten Reaktionsdauer bevorzugt durch Zugabe einer geeigneten Base, als Feststoff oder als wässrige Lösung, z.B. 40 Gew.% wässrige Kaliumcarbonat-Lösung, in einem Bereich von 3 bis 7 gehalten.

Nach bevorzugt 6 h bis 24 h wird nach Zugabe einer weiteren Portion der Verbindung der Formel (II) sowie eines erfindungsgemäßen Reduktionsmittels, z. B. Natriumdithionit, bei bevorzugt -5 °C bis 40 °C, besonders bevorzugt 10 °C bis 25 °C über bevorzugt 3 h bis 24 h, besonders bevorzugt 6 h bis 24 h, mit einer erfindungsgemäßen Verbindung der Formel R²-Y, z.B. Heptafluoro-2-iod-propan versetzt. Der pH Wert der Reaktionsmischung wird dabei während der gesamten Reaktionsdauer bevorzugt durch Zugabe einer geeigneten Base, als Feststoff oder als wässrige Lösung, z.B. wässriger Kaliumcarbonat-Lösung, in einem Bereich von 3 bis 7 gehalten. Nach bevorzugt 6 h bis 24 h kann der Prozess gegebenenfalls erneut wiederholt werden oder die wässrige Phase abgetrennt, die organische Phase gegebenenfalls mit wässriger Natriumchlorid-Lösung, z. B. 20 Gew. %, und/oder wässriger Salzsäure, z. B. 5 Gew. % oder 2,5 Gew.%, und einer wässrigen Natriumchlorid-Lösung, z. B. 10 Gew. %, gewaschen. Somit werden die Produkte aus Schritt (1a) als Lösung in dem organischen Lösungsmittel, z. B. Ethylacetat, erhalten.

Zu einer Lösung aus einer Verbindung der Formel R⁴SO₃H, z. B. Methansulfonsäure, 4-Toluolsulfonsäure oder Benzensulfonsäure, in einem geeigneten organischen Lösungsmittel, z. B. tert Butylmethylether, wird bevorzugt langsam, besonders bevorzugt durch eine kontinuierliche Dosierung über einen bestimmten Zeitraum die Verbindungen des Schritts (1a) als Lösung in dem organischen Lösungsmittel, z. B. Ethylacetat, bei bevorzugt 15 °C bis 25 °C, besonders bevorzugt 20 °C, gegeben, gefolgt durch kontinuierliche Zugabe über einen bestimmten Zeitraum eines weiteren organischen Lösungsmittels, z.B. Heptan, bei bevorzugt 15 °C bis 25 °C, besonders bevorzugt 20 °C. Die Reaktionsmischung wird bevorzugt für 3 h bis 24 h, besonders bevorzugt für 6 h bis 24 h bei einer Temperatur in einem Bereich von bevorzugt 5 °C bis 25 °C, besonders bevorzugt von 15 °C bis 25 °C gerührt und anschließend bevorzugt für 1 h bis 3 h, besonders bevorzugt 1,5 h bis 2,5 h bei bevorzugt 5 °C bis 15 °C, besonders bevorzugt 10 °C, gerührt. Die Verbindungen der Formel (III) fallen als Feststoff in der Reaktionsmischung an und werden anschließend isoliert, bevorzugt durch Filtration. Vorzugsweise wird der Feststoff in einem geeigneten organischen Lösungsmittel, z. B. tert Butylmethylether, bei bevorzugt 15 °C bis 25 °C, besonders bevorzugt 20 °C, suspendiert, erneut isoliert, bevorzugt durch Filtration, und getrocknet.

Die Verbindungen der Formel (III) werden bevorzugt bei -20 °C bis 100 °C, besonders bevorzugt bei 0 °C bis 25 °C, mit einem Halogenierungsmittel, z. B. als Gas, Feststoff, Flüssigkeit, Suspension oder Lösung in einem erfindungsgemäßen organischen Lösungsmittel, z. B. Ethylacetat, über bevorzugt 1 h bis 65 h versetzt. Nach vollständigem Umsatz (HPLC^{a}) wird gegebenenfalls vorhandenes überschüssiges Halogenierungsmittel durch die Zugabe eine Reduktionsmittels, beispielsweise als Reinstoff oder wässrige Lösung, neutralisiert. Die Reaktionsmischung wird gegebenenfalls ein oder mehrfach mit einer alkalischen wässrigen Lösung, z. B. Natriumhydrogencarbonat und/oder einer wässrigen Natriumchloridlösung, z. B. 15 Gew. %, bei bevorzugt 0 °C bis 25 °C gewaschen. Vorzugsweise werden die Waschschritte bei einer Temperatur, die der jeweiligen Reaktionstemperatur entspricht, durchgeführt. Das Lösungsmittel der organischen Phase wird unter vermindertem Druck teilweise entfernt. Vorzugsweise wird die reduzierte organische Phase mit einem weiteren geeigneten Lösungsmittel, z. B. Heptan, versetzt und bevorzugt für 0,5 h bis 3 h, besonders bevorzugt 1 h gerührt und anschließend als Feststoff angefallene Nebenprodukte des Halogenierungsmittels isoliert, bevorzugt durch Filtrieren. Nach Abtrennung des Feststoffs wird die verbleibende organische Phase langsam mit einem oder mehreren geeigneten weiteren Lösungsmittel, z. B. Methanol und Wasser, versetzt und die als Feststoff angefallenen Verbindungen der Formel (I) isoliert, bevorzugt durch Filtrieren. (Schritt (1) (zweifach) und (2))

Eine besonders bevorzugte Ausführungsform des erfindungsgemäßen Verfahrens ist die folgende:
Die Verbindungen der Formel (II) werden in einem Gemisch aus Ethylacetat und Wasser vorgelegt und nach Zugabe von Tetra-n-butylammoniumhydrogensulfat sowie Natriumdithionit, bei 15 °C bis 25 °C, mit Heptafluoro-2-iod-propan versetzt. Der pH Wert der Reaktionsmischung wird dabei während der gesamten Dosier- und Reaktionsdauer durch Zugabe von Kaliumcarbonat in einem Bereich von 4 bis 7 gehalten. Nach bevorzugt 6 h bis 24 h wird die wässrige Phase abgetrennt, die organische Phase zweimal mit einer Mischung aus einer 20 Gew. % wässrigen Natriumchlorid-Lösung und einer 2,5 Gew. % wässrigen Salzsäure und einmal mit einer 10 Gew. % wässrigen Natriumchlorid-Lösung gewaschen. Die Produkte aus Schritt (1a) werden als Lösung in Ethylacetat erhalten.

Zu einer Lösung aus 4-Toluolsulfonsäure in tert-Butylmethylether werden die Verbindungen des Schritts (1a) als Lösung in Ethylacetat langsam bei 15 °C bis 25 °C gegeben, gefolgt durch langsame Zugabe von Heptan bei 15 °C bis 25 °C. Die Reaktionsmischung wird für 6 h bis 24 h bei einer Temperatur in einem Bereich von 15 °C bis 25 °C gerührt und anschließend für weitere 1,5 h bis 2,5 h bei 10 °C gerührt. Die Verbindungen der Formel (III) werden als Feststoff der Reaktionsmischung durch Filtrieren isoliert. Der Feststoff wird in tert Butylmethylether bei 20 °C, suspendiert, erneut filtriert und getrocknet.

Die Verbindungen der Formel (III) werden bei 0 °C bis 25 °C, mit Chlor, N-Chlorsuccinimid (NCS) oder 1,3,5-Trichlor-1,3,5-triazin-2,4,6-trion (TCCA) (Chlorierung) bzw. Brom, N-Bromsuccinimid (NBS) oder 1,3-Dibrom-5-5-dimethylhydantoin (DBDMH) (Bromierung) als Gas, Feststoff, Flüssigkeit, Lösung oder Suspension in Ethylacetat, über bevorzugt 1 h bis 65 h versetzt. Nach vollständigem Umsatz (HPLC^{a}) wird gegebenenfalls vorhandenes überschüssiges Halogenierungsmittel durch die Zugabe von Natriumdithionat als wässrige Lösung neutralisiert. Dazu wird die Reaktionsmischung zweimal mit einer wässrigen Natriumhydrogencarbonat-Lösung, einmal mit einer wässrigen Natriumdithionat-Lösung und einmal mit einer wässrigen 15 Gew. % Natriumchlordlösung bei 0 °C bis 25 °C gewaschen. Die Waschschritte werden bei der Temperatur, die der jeweiligen Reaktionstemperatur entspricht, durchgeführt. Das Lösungsmittel der organischen Phase wird unter vermindertem Druck teilweise entfernt. Die reduzierte organische Phase wird mit Heptan versetzt und für 1 h gerührt und anschließend als Feststoff angefallene Nebenprodukte des Halogenierungsmittels durch Filtrieren isoliert. Die organische Phase wird langsam mit Methanol und anschließend mit Wasser versetzt und die Verbindungen der Formel (I) als Feststoff durch Filtrieren isoliert. (Schritt (1) und (2)).

### Beispiele

Die nachfolgenden Beispiele erläutern das erfindungsgemäße Verfahren näher, ohne die Erfindung dabei auf diese einzuschränken.

### Methoden:

Die NMR-Daten der Beispiele werden in klassischer Form (δ-Werte, Multiplettaufspaltung, Anzahl der H-Atome) aufgeführt.

Das Lösungsmittel und die Frequenz, in welchem das NMR-Spektrum aufgenommen wurde, sind jeweils angegeben.
^{a)} HPLC (High Performance Liquid Chromatography) an einer Phasenumkehrsäule (C18), Waters Arc HPLC-System: Agilent Eclipse Plus C18, 4.6 x 50 mm, 1.8µm oder Äquivalent; Eluent A: Wasser (0.05% TFA); Eluent B: Acetonitril (0.05% TFA); linearer Gradient von 10 % Acetonitril bis 60% Acetonitril in 5 min., dann von 60% Acetonitril bis 80% Acetonitril in 10 min., dann von 80% Acetonitril bis 95% Acetonitril in 2 min.; dann 95% Acetonitril für weitere 2 min; Ofentemperatur 30 °C; Fluss:1,0 mL/min.

### Schritt 1: Herstellung der Verbindungen der Formel (III)

### Tosylat-Salz des 4-[1,2,2,2-Tetrafluoro-1-(trifluoromethyl)ethyl]anilins (III-1)

Schritt (1a): 200 g (1,0 Äquivalente) Anilin, 454,8 g (1,1 Äquivalente) Natriumdithionit, 7,4 g (0,01 Äquivalente) Tetra-n-butylammoniumhydrogensulfat, 268,4 g (0,9 Äquivalente) K₂CO₃ wurden unter Stickstoff in 1500 mL Ethylacetat und 1580 mL Wasser vorgelegt und langsam mit 732,2 g (1,1 Äquivalente) Heptafluoro-2-iod-propan bei 20±2°C versetzt. Nach beendeter Zugabe wurde die Reaktionsmischung für 7 h bei 20±2 °C gerührt bis die HPLC-Analyse einer Probe eine Peakfläche für Anilin von ≤ 0,2 % anzeigte. Die Phasen der Reaktionsmischung wurden bei 20±2 °C voneinander abgetrennt. Die organische Phase wurde zweimal mit einer Mischung aus 260 mL einer 20 Gew. % wässrigen Natriumchlorid-Lösung und aus 260 mL einer 2,5 Gew. % wässrigen Salzsäure-Lösung und einmal mit 260 mL einer 10 Gew. % wässrigen Natriumchlorid-Lösung bei 20±5 °C gewaschen. Die Verbindung 4-[1,2,2,2-Tetrafluoro-1-(trifluoromethyl)ethyl]anilin wurde nach Phasentrenung als Lösung in Ethylacetat erhalten.

Schritt (1b): Zu einer Mischung aus 449,4 g (1,1 Äquivalente) 4-Toluolsulfonsäure Monohydrat in 1000 mL tert-Butylmethylether wurde langsam die Lösung des 4-[1,2,2,2-Tetrafluoro-1-(trifluoromethyl)ethyl]anilins in Ethylacetat bei 20±5 °C hinzugegeben und anschließend langsam 2900 mL Heptan bei 20±5 °C hinzugegeben. Die Mischung wurde über Nacht bei 20±5 °C gerührt, anschließend auf 10 °C abgekühlt, für weitere 2 h bei dieser Temperatur gerührt und filtriert. Der Feststoff wurde in 2200 mL tert-Butylmethylether bei 20 °C suspendiert, filtriert und getrocknet.

Es wurden 789 g (85%) des Tosylat-Salzes der Verbindung 4-[1,2,2,2-Tetrafluoro-1-(trifluoromethyl)ethyl]anilin (III-1) als hellgelber Festoff mit 99,7 % Reinheit (HPLC) erhalten.
¹H-NMR (400 MHz, Methanol-d₄,) δ (ppm) = 7.86 (d, *J* = 8.3 Hz, 2H), 7.72 (d, *J* = 8.1 Hz, 2H), 7.65 (d, *J =* 8.7 Hz, 2 H), 7.24 (d, *J* = 7.9 Hz, 2H), 2.38 (s, 3H). MS [ESI]: Calcd for C₉H₇F₇N [M + H]⁺ 262.0, Gemessen 262.1.

### Schritt (2): Herstellung der Verbindungen der Formel (I)

### 2,6-Dichloro-4-[1,2,2,2-tetrafluoro-1-(trifluoromethyl)ethyl]anilin (I-1a)

### Variante a: Chlorierung mit Chlorgas

Durch eine Lösung von 5,0 g (1.0 Äquivalente) des Tosylat-Salzes des 4-[1,2,2,2-Tetrafluoro-1-(trifluoromethyl)ethyl]anilins (III-1) in 40 mL Ethylacetat wurde bei 0-5 °C Chlorgas geleitet bis die HPLC-Analyse einer Probe eine Peakfläche des 4-[1,2,2,2-Tetrafluoro-1-(trifluoromethyl)ethyl]anilins von ≤ 0,1% und für die entstehende mono-chlorierte Verbindung von ≤ 0,1% anzeigte. Die Reaktionsmischung wurde bei 0-5 °C zweimal mit 10 mL einer wässrigen Natriumhydrogencarbonat-Lösung und einmal mit 10mL einer 15 Gew. % wässrigen Natriumchlorid-Lösung gewaschen. Das Lösungsmittel der organischen Phase wurde unter reduziertem Druck entfernt. Das Rohprodukt wurde in 2,5 mL Methanol aufgenommen und anschließend langsam 20 mL Wasser hinzugegeben, wobei ein Feststoff erhalten wurde, der bei 25 °C unter Stickstoff filtriert und getrocknet wurde.

Es wurden 2,75 g (72,4 %) der Verbindung I-1a als hellgelber Feststoff mit 98,25 % Reinheit (HPLC) erhalten.
¹H-NMR (400 MHz, CDCl₃) δ (ppm) = 7.41 (s, 2H), 4.76 (br s, 2H).

### Variante b: Chlorierung mit N-Chlorsuccinimid

Zu einer Lösung von 74 g (2,4 Äquivalente) NCS in 800 mL Ethylacetat wurden unter Stickstoff 100 g (1,0 Äquivalente) des Tosylat-Salzes des 4-[1,2,2,2-Tetrafluoro-1-(trifluoromethyl)ethyl]anilins (III-1) bei 20 bis 25 °C hinzugegeben. Die Mischung wurde für 64 h bei 20 bis 25 °C gerührt bis die HPLC-Analyse einer Probe eine Peakfläche des 4-[1,2,2,2-Tetrafluoro-1-(trifluoromethyl)ethyl]anilins von ≤ 0,1% und für die entstehende mono-chlorierte Verbindung von ≤ 0,1% anzeigte. Die Reaktionsmischung wurde bei 20-25 °C zweimal mit 100 mL einer wässrigen Natriumhydrogencarbonat-Lösung, einmal mit 100 mL einer wässrigen Natriumdithionat-Lösung und einmal mit 100 mL einer 15 Gew. % wässrigen Natriumchlorid-Lösung gewaschen. Das Lösungsmittel der organischen Phase wurde unter reduziertem Druck teilweise entfernt, 500 mL Heptan hinzugegeben und für 1 h gerührt. Die Mischung wurde filtriert, um das Succinimid zu entfernen. Das Lösungsmittel des Filtrats wurde unter reduziertem Druck teilweise entfernt, 50 mL Methanol hinzugegeben, anschließend langsam mit 400 mL Wasser versetzt, wobei ein Feststoff erhalten wurde, der bei 25 °C unter Stickstoff filtriert und getrocknet wurde.

Es wurden 70,2 g (92 %) der Verbindung I-1a als hellgelber Feststoff mit 99,83 % Reinheit (HPLC) erhalten. Schmelzpunkt: 48 °C.
¹H-NMR (400 MHz, CDCl₃,) δ (ppm) = 7.41 (s, 2H), 4.76 (br s, 2H).

### Umsetzung mit verschiedenen Säuren

Die Umsetzung mit verschiedenen Säuren wurde mit einer Reaktionsmischung des Schritts (1a) ausgehend von Anilin, d.h. mit den Reaktionsprodukten III-1, III-A und III-B, bei 25 °C in Ethylacetat mit verschiedenen Säuren durchgeführt. Dabei wurde der Anteil des gewünschten Salzes der Verbindung III-1 im erhaltenen Feststoff im Vergleich zu den entsprechenden Salzen der Verbindungen III-A und III-B im Feststoff durch HPLC-Analyse bestimmt.

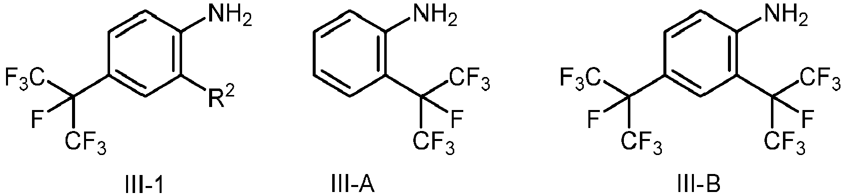

**Tabelle 1: Umsetzung mit verschiedenen Säuren**

| **Säuren** | **HPLC-Analyse** | | | **Feststoffbildung** |
|---|---|---|---|---|
| (Äq. = Äquivalente) | III-1-Salz | III-A-Salz | III-B-Salz | |
| Salzsäure (1.2 Äq.) | 97,7% | 0,4% | 0% | Geringe Feststoffbildung |
| Schwefelsäure (1.2 Äq.) | 99,5% | 0,3% | 0,1% | Geringe Feststoffbildung |
| Essigsäure (0.9 Äq.) | N/A | N/A | N/A | Keine Feststoffbildung |
| Trifluoressigsäure (0.9 Äq.) | 100% | 0% | 0% | Geringe Feststoffbildung |
| Methansulfonsäure (0.9 Äq.) | 99,7% | 0.16% | 0% | Feststoffbildung von III-1 und III-A mit Methansulfonsäure |
| 4-Toluolsulfonsäure•H₂O (0.9 Äq.) | 100% | 0% | 0% | Selektive Feststoffbildung von III-1 mit 4-Toluolsulfonsäre |
| 4-Toluolsulfonsäure•H₂O (1.2 Äq.) | 100% | 0% | 0% | |
| Benzensulfonsäure (0.9 Äq.) | 100% | 0% | 0% | Selektive Feststoffbildung von III-1 mit Benzensulfonsäure |

| | | | | |
|---|---|---|---|---|
| N/A: nicht verfügbar | | | | |

Bei der Verwendung von anorganischen Säuren, z.B. Mineralsäuren wie Salzsäure und Schwefelsäure wurde bei Zugabe eines Überschusses bis zu 1,2 Äquivalenten nur eine geringeAusbeute an Verbindung III-1 erhalten. Dabei wurden Salze der Verbindungen III-1, III-A und auch von III-B durch HPLC-Analyse nachgewiesen. Die Kristallisation erfolgte zudem nicht in ausreichend hoher Reinheit, da die auskristallisierten Feststoffe neben der Verbindung III-1 auch Anteile an Verbindung III-A bzw. an Verbindung III-B enthielten.

In allen Beispielen der Umsetzung mit einer Verbindung der Formel R⁴SO₃H wurde ein hoher Anteil an Feststoff erhalten. Die Umsetzung mit einer Verbindung der Formel R⁴SO₃H verlief mit hoher Ausbeute.

Bei Verwendung der Methansulfonsäure wurde ein hoher Anteil an Feststoff der Mesylat-Salze erhalten. Dabei wurden durch HPLC-Analyse lediglich die Mesylat-Salze der Verbindungen III-1 und III-A, jedoch nicht der Verbindung III-B nachgewiesen.

Die Verwendung von 4-Toluolsulfonsäure führte unabhängig von den eingesetzten Äquivalenten zu einer selektiven Bildung des Tosylat-Salzes der Verbindung III-1. Durch HPLC-Analyse wurden kein Tosylat-Salz der Verbindungen III-A oder III-B nachgewiesen. Bei Verwendung von 4-Toluolsulfonsäure wurde ein hoher Anteil an Feststoff des Tosylat-Salzes erhalten. Die Umsetzung mit 4-Toluolsulfonsäure verlief somit unabhängig von den eingesetzten Äquivalenten mit hoher Selektivität (100 %), hoher Reinheit und mit hoher Ausbeute an Feststoff. Ausgehend von Anilin (200 g) als eine Verbindung der Formel (II) wurde das Tosylat-Salz der Verbindung III-1 mit 85 % Ausbeute, 100 % Selektivität und einer Reinheit von 99,7 % (HPLC) erhalten. Die Tosylat-Salze der Verbindungen III-1 sind somit zum Einsatz in einem GMP-regulierten Umfeld geeignet. Weiterhin ist vorteilhaft, dass 4-Toluolsulfonsäure ein kostengünstiges Reagenz ist und damit ein kosteneffektives Verfahren ermöglicht.

Auch bei Verwendung von Benzensulfonsäure wurde eine hohe Selektivität für die Bildung des Besilat-Salzes der Verbindung III-1 (100 %) erreicht. Durch HPLC-Analyse wurde kein Besilat-Salz der Verbindungen III-A oder III-B nachgewiesen. Auch wurde ein hoher Anteil an Feststoff des Besilat-Salzes erhalten.

Die Umsetzung mit einer Verbindung der Formel R⁴SO₃H in Schritt (1b) verlief somit mit hoher Ausbeute, hoher Selektivität und hoher Reinheit. Die Verbindungen der Formel (III) konnten mit hoher Produktqualität auch in großtechnischem Maßstab auf einfache Weise isoliert werden. Die Bildung unerwünschter Nebenprodukte wurde optimiert. Aufwendige und kostenintensive Aufarbeitungsschritte sowie kostenintensive Materialien zur Aufarbeitung entfielen. Der Einsatz von Ausgangsmaterialien, Reagenzien und der Abfallströme wurde reduziert und somit die Umweltfreundlichkeit des Verfahrens erhöht. Da die Verbindungen der Formel R⁴SO₃H kostengünstig und auch in großem Maßstab bereit gestellt werden konnten, wurde ein kostengünstiges Verfahren ermöglicht.

## Patentansprüche

1. Verfahren zur Herstellung von Verbindungen der Formel (I) 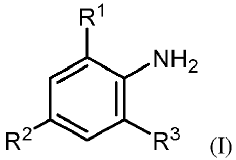 worin
R¹ für Chlor oder Brom steht,
R² für C₁-C₄-Halogenalkyl steht und
R³ für Cyano, Halogen, gegebenenfalls mit Halogen oder CN substituiertes C₁-C₄-Alkyl oder für gegebenenfalls mit Halogen substituiertes C₁-C₄-Alkoxy steht,
ausgehend von Verbindungen der Formel (IIa), 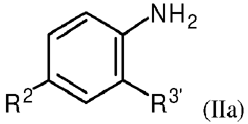 worin R² die oben genannte Bedeutung hat und
R^{3'} für Wasserstoff, Cyano, Halogen, gegebenenfalls mit Halogen oder CN substituiertes C₁-C₄-Alkyl oder für gegebenenfalls mit Halogen substituiertes C₁-C₄-Alkoxy steht,
umfassend die folgenden Schritte (1b) und (2)
(1b) Umsetzung von Verbindungen der Formel (IIa) mit einer Verbindung der Formel R⁴SO₃H zu Verbindungen der Formel (III) oder mit einer aromatischen Carbonsäure R⁵CO₂H zu Verbindungen der Formel (IIIa), 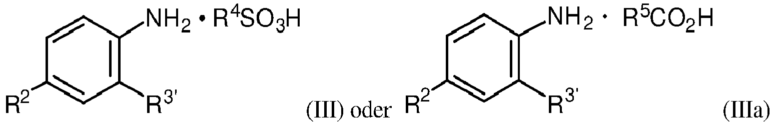 wobei R² und R^{3'} die oben genannten Bedeutungen haben und R⁴ ein gegebenenfalls substituiertes C₁-C₁₂-Alkyl, das verzweigt oder unverzweigt sein kann, oder ein gegebenenfalls substituierter 6- bis 14-gliedriger Aromat ist und R⁵ ein gegebenenfalls substituierter 6-gliedriger Aromat, insbesondere C₆H₅, C₆H₄Cl oder C₆H₄NO₂, ist und
(2) Chlorierung oder Bromierung von Verbindungen der Formel (III) oder (IIIa) mit einem Chlorierungs- oder Bromierungsmittel zu Verbindungen der Formel (I).

2. Verfahren gemäß Anspruch 1, wobei die Verbindungen der Formel (IIa) in einem Schritt (1a) hergestellt werden durch Umsetzung von Verbindungen der Formel (II), 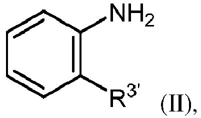 worin R^{3'} dieselbe Bedeutung wie in Anspruch 1 definiert hat, mit Verbindungen der Formel R²-Y, wobei Y für Iod oder Brom steht und R² dieselbe Bedeutung wie in Anspruch 1 definiert hat.

3. Verfahren gemäß einem der vorangehenden Ansprüche, wobei die Umsetzung mit einer Verbindung der Formel R⁴SO₃H oder mit einer aromatischen Carbonsäure R⁵CO₂H in Schritt (1b) ein Salz liefert, vorzugsweise fällt das Salz als Feststoff aus.

4. Verfahren gemäß einem der vorangehenden Ansprüche, **dadurch gekennzeichnet, dass** das Chlorierungs- oder Bromierungsmittel in Schritt (2) ausgewählt ist aus Chlor, Brom, *N*-Chlorsuccinimid (NCS), *N*-Bromsuccinimid (NBS), 1,3-Dichlor-5-5-dimethylhydantoin (DCDMH), 1,3-Dibrom-5-5-dimethylhydantoin (DBDMH), 1,3,5-Trichlor-1,3,5-triazin-2,4,6-trion, 1,3,5-Tribrom-1,3,5-triazin-2,4,6-trion oder 1,3-Dibrom-1,3,5-triazin-2,4,6-trion.

5. Verfahren gemäß einem der vorangehenden Ansprüche, **dadurch gekennzeichnet, dass** in Schritt (1a) ein organisches Lösungsmittel, das ausgewählt ist aus Ethylacetat, Isopropylacetat, tert-Butylmethylether, Cyclopentylmethylether, Methyl-THF oder Mischungen davon, eingesetzt wird, insbesondere zusammen mit Wasser.

6. Verfahren gemäß einem der vorangehenden Ansprüche, **dadurch gekennzeichnet, dass** in Schritt (1b) ein organisches Lösungsmittel, das ausgewählt ist aus Methylacetat, Ethylacetat, Isopropylacetat, tert-Butylmethylether, Cyclopentylmethylether, THF oder Methyl-THF oder Mischungen davon, eingesetzt wird.

7. Verfahren gemäß einem der vorangehenden Ansprüche, **dadurch gekennzeichnet, dass** in Schritt (1b) die Verbindungen der Formel (III) oder (IIIa) gefällt werden, wobei ein Antisolvens zugegeben wird, insbesondere Hexan, Heptan, Methylcylohexan oder Cyclohexan, dabei insbesondere Heptan oder Methylcyclohexan.

8. Verfahren gemäß einem der vorangehenden Ansprüche, **dadurch gekennzeichnet, dass** in Schritt (2) ein organisches Lösungsmittel, das ausgewählt ist aus Acetonitril, Ethylacetat, Isopropylacetat, tert-Butylmethylether, Cyclopentylmethylether, Methyl-THF, insbesondere 2-Methyl-THF, Aceton, Methylethylketon, Methyl-tert-butylketon, Methyl-isobutylketon, Alkane wie Hexan, Heptan, Methylcyclohexan oder Cyclohexan oder Mischungen davon eingesetzt wird.

9. Verfahren gemäß einem der vorangehenden Ansprüche, **dadurch gekennzeichnet, dass** in den Schritten (1a), (1b) und Schritt (2) dasselbe organische Lösungsmittel oder Mischungen davon eingesetzt wird.

10. Verfahren gemäß einem der vorangehenden Ansprüche, **dadurch gekennzeichnet, dass** R² für mit Fluor substituiertes C₁-C₄-Alkyl, vorzugsweise für Perfluoro-C₁-C₃-Alkyl steht.

11. Verfahren gemäß einem der vorangehenden Ansprüche, **dadurch gekennzeichnet, dass** R³ für Cl, Br, C₁-C₃-Alkyl oder mit Fluor substituiertes C₁-C₃-Alkyl, C₁-C₃-Alkoxy oder mit Fluor substituiertes C₁-C₃-Alkoxy steht und R^{3'} für Wasserstoff, Cl, Br, C₁-C₃-Alkyl oder mit Fluor substituiertes C₁-C₃-Alkyl, C₁-C₃-Alkoxy oder mit Fluor substituiertes C₁-C₃-Alkoxy steht.

12. Verfahren gemäß einem der vorangehenden Ansprüche, **dadurch gekennzeichnet, dass** R⁴ ein gegebenenfalls mit Halogen, Cyano oder Hydroxy substituiertes C₁-C₁₂-Alkyl, das verzweigt oder unverzweigt sein kann, ist oder ein 6- bis 14-gliedriger Aromat, der gegebenenfalls mit Halogen, Cyano oder Hydroxy oder mit einem gegebenenfalls mit Halogen, Cyano oder Hydroxy substituiertem C₁-C₁₂-Alkyl, das verzweigt oder unverzweigt sein kann, substituiert ist.

13. Verfahren gemäß einem der vorangehenden Ansprüche, **dadurch gekennzeichnet, dass** R⁴ für Methyl, Ethyl, n-Propyl, iso-Propyl, n-Butyl, iso-Butyl, sek-Butyl, tert-Butyl, n-Pentyl, iso-Pentyl, Neopentyl, tert-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl, n-Dodecyl, Phenyl, Tolyl, 4-Tolyl, Naphthyl, Anthryl oder Phenanthrenyl.

14. Verfahren gemäß einem der vorangehenden Ansprüche, **dadurch gekennzeichnet, dass** die Verbindung der Formel R⁴SO₃H Methansulfonsäure, 4-Toluolsulfonsäure oder Benzensulfonsäure ist.

15. Verfahren gemäß einem der vorangehenden Ansprüche, **dadurch gekennzeichnet, dass** die Umsetzung mit einer Verbindung der Formel R⁴SO₃H erfolgt,
R¹ für Chlor oder Brom,
R² für Heptafluor-iso-propyl,
R³ für Chlor, Trifluormethyl, Trifluormethoxy oder Difluormethoxy,
R³ für Wasserstoff, Chlor, Trifluormethyl, Trifluormethoxy oder Difluormethoxy
und
R⁴ für 4-Tolyl steht.
